# FASCICULE DE BREVET EUROPEEN

(11) **EP 1 514 884 B1**
(45) Date de publication et mention de la délivrance du brevet: **26.08.2009**
(21) Numéro de dépôt: 04104372.0
(22) Date de dépôt: 10.09.2004
(51) Int. Cl.: B29C 49/00, B32B 27/32, C08J 5/18, C08L 23/08

(54) **Copolymères éthyléniques séquences comprenant une séquence vinyllactame, compositions cosmétiques ou pharmaceutiques les contenant, et utilisation de ces copolymères en cosmétique**
Blockcopolymere enthaltend einen Vinyllactamblock, kosmetische Zusammensetzung und Verwendung dieser Polymere in der Kosmetik
Block copolymer comprising a vinyllactam block, cosmetic composition and use of this polymer in cosmetics

(30) Priorité: 15.09.2003 FR 0350537
(43) Date de publication de la demande: 16.03.2005
(73) Titulaire: L'ORÉAL, 75008 Paris (FR)
(72) Inventeur: MOUGIN, Nathalie, 75011, PARIS (FR)
(74) Mandataire: Poulin, Gérard

(56) Documents cités:
- EP-A- 1 510 533
- FR-A- 2 327 761
- US-A- 3 739 042
- US-B1- 6 369 165

## Description

### DOMAINE TECHNIQUE

La présente invention a trait à de nouveaux polymères de structure spécifique de type copolymère éthylénique séquence comprenant une séquence vinyllactame.

La présente invention concerne, en outre, une composition, notamment cosmétique ou pharmaceutique en particulier une composition capillaire, comprenant ledit polymère de structure spécifique.

L'invention a également trait à l'utilisation de ces polymères en cosmétique pour le traitement des matières kératiniques, notamment de la peau, des ongles ou des cheveux.

Précisons que par "séquence vinyllactame", on entend une séquence comprenant un motif lactame, par exemple des dérivés de lactames, en d'autres termes, de manière générale, une séquence susceptible d'être préparée par polymérisation d'un monomère à cycle lactame.

De nombreuses compositions cosmétiques, et en particulier les compositions capillaires, dites compositions de "hair styling", qui se présentent sous la forme d'aérosols ("sprays") de gels, de mousses ou de shampooings contiennent des résines ou polymères.

Il s'agit, en particulier, de polymères acryliques ayant des températures de transition vitreuse (Tg) élevées, tels que ceux décrits dans le document FR-A-2 439 798.

De tels polymères, apportent, notamment en coiffage, un maintien de la chevelure, mais ils présentent l'inconvénient d'une trop grande friabilité, ce qui ne permet pas une bonne tenue dans le temps de la chevelure.

Dans le cas des vernis, les polymères existants ne résistent pas aux chocs.

Pour résoudre les problèmes posés par ces ²polymères, on utilise, en outre, dans les compositions cosmétiques, des plastifiants, afin d'abaisser la température de transition vitreuse. Mais, alors, les polymères tendent à présenter des effets de « collants » ou, dans le cas du coiffage ne permettent pas de fixation « ultra-forte ».

En outre, lorsque les cheveux sont coiffés, nombreux sont les polymères de coiffage ("hair styling") existant qui forment des particules blanches, ce qui n'est pas acceptable, en particulier lorsque les cheveux sont bruns et/ou épais. D'autres inconvénients présentés par les polymères utilisés à l'heure actuelle sont leur incompatibilité avec les propulseurs pour aérosols existant.

On connaît aussi, par ailleurs, dans le domaine de la cosmétique des polymères à base de vinyllactames par exemple de vinylpyrrolidone, et en particulier l'homopolyvinylpyrrolidone.

Le principal défaut de ces polymères est leur forte hygroscopie, qui conduit à leur donner en présence de l'humidité ambiante, un fort caractère collant.

Ainsi, le document EP-A-1 002 811 de BASF décrit des polymères greffés solubles dans l'eau ou dispersibles dans l'eau obtenus par polymérisation radicalaire de monomères essentiellement acryliques, et d'un prépolymère polymérisable à base de vinyllactame par exemple de vinylpyrrolidone ou de vinylcaprolactame.

Ces polymères sont utilisés notamment dans des compositions capillaires.

Le document US-A-6 193 961 de ISP décrit un terpolymère homogène de N-vinyllactame de préférence de N-vinylpyrrolidone ou de N-vinyl-caprolactame, d'acrylate de diméthylaminoalkyle ou de diméthylaminoakylacrylamide et d'un monomère polysiloxane.

Ces terpolymères sont utilisés dans des compositions cosmétiques et de soins par exemple des compositions cosmétiques telles que des gels coiffants ou des mousses.

Dans ce document sont cités de nombreux autres brevets mentionnant l'utilisation de polymères à base de vinyllactame dans les compositions de soins pour la peau et les cheveux, tels que les documents US-A-3 914 403, US-A-3 954 960, US-A-4 039 734, US-A-4 057 533, US-A-4 210 161, US-A-4 223 009, US-A-4 586 518, US-A-4 764 363, US-A-4 834 968, US-4 842 850, US-A-4 902 499, US-A-4 906 459, US-A-4 923 694, US-4 963 348, US-A-5 011 895, US-A-5 015 708, US-A-5 126 124, US-A-5 158 762, US-A-5 275 809, US-A-5 502 136, WO-A-91/15186, WO-A-91/15185, EP-A2-412704, EP-A1-0412707 et JP-A-57126409.

Dans ce même document, on indique également que de nombreux brevets décrivent l'utilisation, en particulier, d'une N-vinyllactame dans le domaine de la cosmétique et des produits pharmaceutiques, en particulier dans les aérosols pour les cheveux. Ces brevets sont les documents US-A-3 910 862, US-A-4 923 694, US-A-5 045 617, US-A-5 321 110, US-A-5 492 988 et US-A-5 637 296.

Ainsi, le document US-A-3 954 960 a trait à des compositions cosmétiques et capillaires qui contiennent en tant que résine filmogène un copolymère quaternisé de vinylpyrrolidone et d'un monomère vinylique copolymérisable à savoir un méth(acrylate) de dialkylaminoalkyle.

Le brevet US-A-3 914 403, quant à lui, est relatif à des compositions capillaires qui contiennent un mélange de résines filmogènes d'un homo ou copolymère de N-vinylpyrrolidone en mélange avec un copolymère quaternisé de vinylpyrrolidone et d'un monomère vinylique copolymérisable avec celle-ci à savoir un méth(acrylate) de dialkylaminoalkyle.

Le document US-A-5 502 136 concerne un procédé de préparation de copolymères de vinylpyrrolidone et d'acétate de vinyle par polymérisation radicalaire.

Le document WO-A-00/68282 a trait à des terpolymères à base de vinylpyrrolidone (VP), de diméthylaminopropyl méthacrylamide (DMAPMA), et du dérivé quaternisé par une chaîne alkyle en C₈ à C₂₄ du DMAPMA, et aux compositions capillaires et cosmétiques les comprenant.

On note que dans ces copolymères des chaînes grasses sont insérées afin de diminuer le collant et d'augmenter la résistance à l'humidité, mais la gamme des propriétés des polymères obtenus se trouve ainsi limitée. Par ailleurs, aucune valeur du collant « tack » de ces copolymères n'est mentionnée.

Le document de Matyjasezwski et al., Prepr. 38(1), 1997, p. 695 décrit des copolymères à squelette N-vinylpyrrolidone et greffons PDMS pour des hydrogels.

Le brevet WO 97/18247 du même K. Matyjaszewski décrit, à la page 103, un exemple d'homopolymère de vinylpyrrolidone.

Le document FR-A-2 327 761 est relatif à des compositions cosmétiques comprenant un polymère résultant de la polymérisation, en présence de cérium, d'un monomère insaturé sur un polyvinylpyrrolidone diol. Un polymère PVP-poly(méthacrylate de lauryle) est mentionné) L'utilisation d'un copolymère vinylpyrrolidone/acrylate de glucosamine est également décrite. Les polymères de ce document sont des polymères très particuliers, généralement à structure branchée.

Ces polymères apportent brillance et tenue dans le temps à la coiffure.

Mais ils confèrent aussi une certaine rigidité à la coiffure causant un aspect non-naturel. Ils présentent en outre l'inconvénient d'une forte hygroscopie de par la présence de motifs hydroxy n'ayant pas réagi, tout au long des chaînes de polymères.

Le document EP-A1-1 510 533 qui est un document selon l'article 54(3) CBE décrit un copolymère, éthylénique, séquencé, linéaire comprenant au moins une séquence A comprenant de 52 à 100% en poids d'un monomère lactame de formule (I) ; ladite séquence représentant de 1 à 99% en poids du copolymère et au moins une séquence B dont la température de transition vitreuse est de -55°C à +55°C.

La séquence A du copolymère de ce document peut être un copolymère comprenant, outre le monomère lactame de formule (I), un ou plusieurs autres monomères dont on ne précise en aucune manière s'ils sont « non hydrophiles » ou pas.

En outre, une plage restreinte de 60 à 80% en poids de la séquence A pour le monomère éthylénique à cycle lactame n'est en aucun cas mentionnée dans le document EP-A1-1 510 533.

Il existe donc un besoin pour un polymère, qui, lorsqu'il est inclus dans une composition, en particulier une composition cosmétique, fasse en sorte que cette composition ne présente pas les inconvénients, défauts, limitations et désavantages des compositions de l'art antérieur.

Il existe, en particulier, un besoin pour un polymère et une composition le contenant, qui présente une combinaison optimale des propriétés de rigidité et de « collant ».

Ainsi, une composition capillaire comportant le polymère doit permettre d'obtenir plus de tenue, tout en conservant un effet naturel. Le polymère doit notamment, dans de telles compositions montrer de bonnes propriétés de coiffage "styling" et, lors du démêlage, ne pas poudrer, c'est-à-dire former des résidus visibles « flaking ». Par ailleurs, le polymère doit être compatible avec les gaz propulseurs aérosols.

Dans le cas du maquillage des ongles, l'obtention d'un film brillant est souhaité, ce film devant en plus résister aux agressions mécaniques. Le polymère contenu dans la formule doit donc être capable de résister de manière excellente à l'abrasion mécanique.

Dans le cas d'un traitement de la peau, le maquillage, mis en oeuvre, et qui inclut le polymère, doit adhérer à la peau, sans tirer celle-ci tout en étant confortable (ne pas provoquer des « tiraillements »).

Dans tous les cas et quelle que soit la composition dans laquelle se trouve utilisé le polymère, il est nécessaire que ce dernier donne un produit non collant au toucher, en particulier en fonction de l'humidité, et notamment dans des conditions d'humidité élevées (par exemple pour des humidités relatives (HR) de 50% à 100%) .

En d'autres termes, il existe un besoin pour un polymère de vinyllactame ne possédant pas, ou peu, de collant ou « tack » et de toute façon un collant ou tack, avec l'humidité, diminué par rapport aux polymères de vinyllactame de l'art antérieur.

Le but de la présente invention est de fournir un polymère qui réponde, entre autres, aux besoins, critères et exigences cités plus haut et qui résolve les problèmes des polymères de l'art antérieur.

Ce but et d'autres encore sont atteints, conformément à la présente invention, par un copolymère éthylénique, séquencé, linéaire comprenant :
- au moins une séquence A susceptible d'être préparée à partir de monomères, constitués en majorité d'un monomère éthylénique à cycle lactame répondant à la formule (I) suivante : 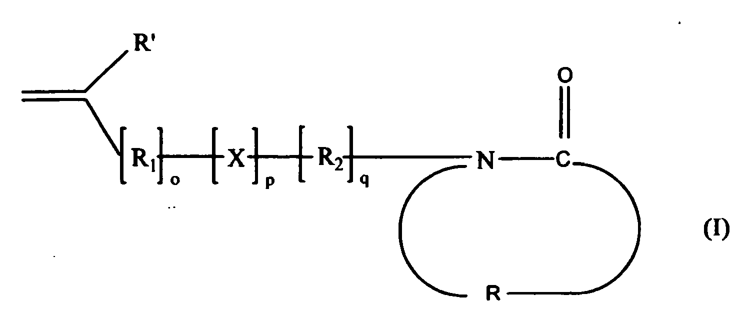 dans laquelle :
   - R représente un groupe -(CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
   - R' représente H ou un groupe méthyle ;
   - R₁ et R₂, qui peuvent être identiques ou différents représentent un groupe alkylène ou aralkylène linéaire, ramifié, ou cyclique, de 1 à 22 C, dans lequel un ou plusieurs des atomes de carbone sont éventuellement remplacés par un atome d'oxygène ou d'azote ;
   - X est choisi parmi -OCO-, -NHCO-, -COO-, -O- ;
   - o, p, q, représentent chacun indépendamment les uns des autres 0 ou 1 ;
   et d'au moins un monomère non hydrophile, à savoir un monomère dont l'homopolymère préparé à partir de ce monomère n'est pas hydrosoluble ou hydrodispersible, le pourcentage de monomère éthylènique à cycle lactame de formule (I) dans la séquence A étant de 60 à 80% en poids;
- et au moins une séquence B susceptible d'être préparée à partir de monomères ne comportant pas de monomère éthylénique à cycle lactame de formule (I) ou comportant une proportion minoritaire de celui-ci.

La teneur (en poids) en monomère éthylénique à cycle lactame est donnée par rapport au poids total des monomères à partir desquels est susceptible d'être préparée la séquence A.

En d'autres termes, la teneur en motifs issus d'un monomère éthylénique à cycle lactame de la séquence A est de 60 à 80% en poids.

Cette teneur en poids est donnée par rapport au poids total de la séquence A.

De préférence, dans la formule (I), o = 0, p = 1, q = 1, R₂ représente -CH₂CH₂-, X représente COO ou CONH, et R est (CH₂) ou (CH₂) ou CH₂CH₂NH.

Le monomère de formule (I) sera de préférence un méthacrylate ou acrylate de pyrrilidinoéthyle, ou un acrylate ou méthacrylate d'uréïdo éthyle.

De préférence, la séquence A est susceptible d'être préparée à partir de monomères, constitué(s) de 60 à 80% en poids d'un vinyllactame, répondant à la formule (II) suivante : 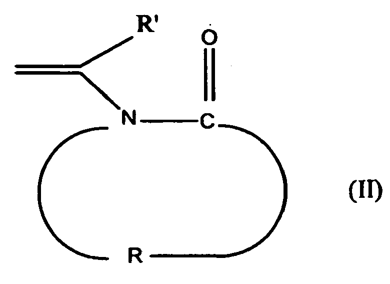 dans laquelle R, R' ont la signification déjà donnée plus haut.

De préférence dans les formules (I) et (II) ci-dessus, R₁ réprésente -(CH₂)ₙ- avec n représentant un entier de 3 à 5, ou bien R est -CH₂-CH₂-NH-.

Avantageusement, le N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

Les N-vinyllactames préférés sont la N-vinylpyrrolidone, la N-vinylcaprolactame et la vinylimidazolidinone.

L'invention a également pour objet les compositions cosmétiques ou pharmaceutiques comprenant lesdits copolymères éthyléniques, séquences, linéaires.

Lorsqu'ils sont incorporés dans de telles compositions, les copolymères présentant la structure spécifique, selon l'invention, permettent d'obtenir des propriétés, ou plutôt une combinaison de propriétés extrêmement intéressantes, qu'il n'était pas possible d'obtenir avec les polymères de l'art antérieur.

De manière générale, les copolymères selon l'invention présentent, du fait de leur structure particulière une hygroscopie réduite par rapport aux copolymères de l'art antérieur présentant des motifs vinyllactames.

Les copolymères de l'invention ont une combinaison optimale de rigidité et de caractère non collant et ils conduisent ainsi à des compositions ou systèmes ayant notamment des résistances mécaniques, des résistances à l'usure et des tenues dans le temps améliorées, et une fragilité réduite, tout en n'étant pas collants.

Les copolymères selon l'invention peuvent donc être définis comme des polymères ne présentant pas de collant ou « tack », qui sont, de préférence, des polymères filmogènes.

Ainsi, lorsque les copolymères, selon l'invention, sont utilisés dans des compositions pour le traitement de la chevelure, telles que des laques, ils apportent plus de tenue dans le temps. Ils sont moins fragiles qu'une laque classique et simultanément ils ne sont pas collants. Le phénomène de poudrage sur les cheveux, observé hors du démélage, avec les compositions de l'art antérieur est évité.

Dans le cas des vernis à ongles, la formule comprenant le copolymère selon l'invention, a une résistance à l'usure supérieure et ne colle pas, tout en adhérant sur l'ongle. De ce fait, la perte de brillance, c'est-à-dire la matification du film par le marquage mécanique ou par le marquage par les poussières qui se produit avec des films collants de l'art antérieur ne se produit pas avec les vernis et films comprenant le copolymère selon l'invention. En effet, l'absence de collant, « tack », de ces derniers fait qu'ils ne sont pas marqués mécaniquement et qu'ils ne retiennent pas les poussières et qu'ils ne subissent donc pas de modification ou perte de brillance.

Les vernis, comprenant le copolymère selon l'invention, donnent des films qui ne s'écaillent pas, grâce notamment à la présence dans le copolymère selon l'invention d'une séquence A susceptible d'être préparée à partir de monomères comprenant, outre le monomère éthylénique à cycle lactame, tel qu'un vinyllactame, à raison de 60 à 80% en poids un ou plusieurs autres monomères qui, spécifiquement, ne sont pas hydrophiles, cette structure particulière du copolymère de l'invention et, en particulier, de la séquence A, empêche l'écaillage des films, mais simultanément et de manière étonnante ne conduit pas à un film collant.

Dans les produits de maquillage, comme les rouges à lèvres ou les fonds de teint, le maquillage présente une bonne tenue sur les lèvres ou la peau, sans laisser de sensation de collant.

L'invention concerne également un procédé cosmétique de maquillage ou de soin des matières kératiniques comprenant l'application sur les matières kératiniques d'une composition selon l'invention.

L'invention concerne donc, en outre, l'utilisation des copolymères selon l'invention pour améliorer le pouvoir coiffant et/ou la tenue de la coiffure, sans collant, notamment au toucher, d'une laque pour cheveux ; l'utilisation des copolymères selon l'invention pour améliorer l'adhérence et la résistance à l'usure, sans collant, notamment au toucher d'un vernis à ongles ; l'utilisation des copolymères selon l'invention pour améliorer la tenue d'une composition de maquillage ; l'utilisation des copolymères selon l'invention pour diminuer le collant, notamment au toucher, d'une composition cosmétique, en particulier dans des conditions d'humidité élevée par exemple pour des HR de 50% à 100% ; et enfin, l'utilisation des copolymères selon l'invention dans une composition cosmétique telle qu'une composition de soin ou de maquillage pour masquer les rides, qui donne à la peau un aspect lissé, sans tiraillement.

Les copolymères, selon l'invention, apportent donc une solution aux problèmes posés par les polymères de l'art antérieur.

Les propriétés avantageuses, inattendues, des copolymères spécifiques de l'invention, qui sont fondamentalement des copolymères linéaires, proviennent notamment, voire essentiellement, de la nature spécifique des séquences qui les constituent et, en particulier, de la séquence A, comprenant de 60 à 80% en poids en monomère éthylénique à cycle lactame, tel qu'un vinyllactame.

De manière étonnante, les copolymères selon l'invention, grâce, semble-t-il, à la présence dans la séquence A, comprenant de 60 à 80% en poids, en monomère éthylénique à cycle lactame, tel qu'un vinyllactame, d'un ou plusieurs autres monomères non hydrophiles ont une hygroscopie réduite par rapport aux polymères à base de vinyllactame de l'art antérieur, et notamment les copolymères de l'invention ont une hygroscopie très fortement réduite par rapport aux homopolymères de vinyllactame de l'art antérieur.

Mais, les copolymères de l'invention ont aussi de manière surprenante, une hygroscopie réduite par rapport aux copolymères de l'art antérieur représenté par exemple par le document FR-A-2 327 761 présentant une structure séquencée, formée d'une part de séquences constituées d'homopolymères de vinyllactame (par exemple, de polyvinylpyrrolidone PVP), et d'autre part de séquences préparées à partir de monomères non hydrophiles, tels que le méthacrylate de méthyle.

Il s'est en effet avéré, de manière étonnante, que l'incorporation du monomère non hydrophile au sein même de la séquence de monomère éthylénique à cycle lactame, tel qu'un vinyllactame, qui se présente donc, selon l'invention, comme un copolymère alterné, à gradient ou statistique du monomère éthylénique à cycle lactame et du monomère non hydrophile, conduisait à une diminution significative de l'hygroscopie par rapport à un copolymère, dans lequel le monomère non hydrophile, est incorporé sous la forme de séquences ou blocs dans le copolymère qui comprend, en outre, des séquences de polyvinyllactame homopolymère.

Le copolymère selon l'invention est caractérisé, en outre, par le fait qu'il comporte, outre la séquence A, préparée à partir de 60 à 80% en poids de monomère éthylénique à cycle lactame, tel qu'un vinyllactame, une autre séquence B, exempte de monomère éthylénique à cycle lactame tel qu'un vinyllactame, ou dans laquelle le monomère éthylénique à cycle lactame est en minorité. Les copolymères selon l'invention présentent, aussi, de ce fait, une diminution de leur caractère hygroscopique et collant par rapport à des copolymères de l'art antérieur qui sont des copolymères statistiques de vinyllactame, par exemple de vinylpyrrolidone et d'un monomère hydrophobe, et qui ne comportent donc pas de séquence B selon l'invention.

De tels copolymères statistiques de l'art antérieur qui sont, par exemple décrits dans le document WO-A-00 68282, cité plus haut, ne permettent pas d'obtenir une diminution du collant aussi importante que celle obtenue avec les copolymères de l'invention, et, en outre, ils ne peuvent présenter qu'un nombre de propriétés restreintes, réduites, du fait de la mise en oeuvre d'un monomère non hydrophile ou hydrophobe très particulier.

Ceci limite la palette de leurs applications éventuelles. Au contraire, les copolymères selon l'invention peuvent présenter des structures très variés du fait de leur structure séquence avec une séquence B et donc conduire à des propriétés adaptées à l'application.

La séquence B peut être choisie à volonté pour communiquer au copolymère selon l'invention toutes les propriétés souhaitables voulues - outre le caractère non collant - qui correspondent à des applications spécifiques. Par exemple, la séquence B pourra être choisie de manière à contrôler, la flexibilité du copolymère et obtenir de manière surprenante des copolymères qui ne sont pas fragiles, friables, et qui donnent des films sans écailles, tout en n'étant pas collants.

Rien ne laissait supposer dans l'art antérieur qu'en mettant en oeuvre un copolymère spécifiquement linéaire, en imposant :
- qu'au moins une séquence (A) de ce copolymère soit constituée de 60 à 80% en poids de monomère éthylénique à cycle lactame tel qu'un vinyllactame ;
- que le ou les autres monomères de cette séquence (A) soient spécifiquement non hydrophiles ; et, enfin,
- qu'au moins une autre séquence (B), ait une structure différente de la séquence (A) et soit exempte de ou constituée en minorité de monomère éthylénique à cycle lactame, tel qu'un N-vinyllactame,
on pourrait parvenir, selon l'invention, à une diminution sans précédents de l'hygroscopie, du caractère collant du copolymère et à obtenir, en choisissant la séquence B voulue, une combinaison de propriétés excellentes pour ce copolymère. La structure spécifique du copolymère de l'invention conduit à une optimisation de ses propriétés, conduisant à un parfait équilibre entre, par exemple, les propriétés de résistance mécanique, et de non-collant.

Sans vouloir être lié par aucune théorie, les propriétés avantageuses du copolymère selon l'invention proviendraient du fait que la nature des séquences est spécifiquement choisie de façon à favoriser la séparation des phases entre celles-ci et donc, entre autres, à contrôler de manière optimale l'hygroscopie et donc le collant du copolymère. Par ailleurs, le fait que le polymère soit linéaire implique une synthèse beaucoup plus facile et contrôlée, ce qui permet de prévoir de façon précise la structure des polymères obtenus, et ainsi d'optimiser les propriétés finales des polymères.

De manière plus précise, les copolymères selon l'invention sont des copolymères séquences ou blocs. On entend généralement par ces termes que les copolymères sont constitués de séquences ou blocs accrochés les uns aux autres de façon covalente.

En outre, deux séquences successives sont de natures différentes. Par contre, deux séquences A ou B non successives peuvent être de même nature.

Chaque séquence A est constituée par un copolymère du monomère éthylénique à cycle lactame et du ou des monomère(s) non-hydrophile(s).

Chaque séquence B peut être constituée d'un homopolymère ou d'un copolymère. Le copolymère constituant la séquence A et éventuellement la séquence B peut être statistique, alterné ou encore à gradient.

Le polymère peut comporter, en outre, une autre séquence C différente des séquences A et B et éventuellement encore d'autres séquences, par exemple une séquence D différente de A, B et C.

Le polymère selon l'invention pourra donc être choisi parmi les copolymères biséquencés de type AB, les copolymères triséquencés de type ABA, BAB, ABC, ACB, avec C différent de A ou B.

Le polymère selon l'invention pourra aussi être choisi parmi les copolymères multiséquencés ayant plus de trois séquences différentes ou non : (AB)ₙ, (ABA)ₙ, (BAB)ₙ, (ABC)ₙ, (ACB)ₙ, avec C différent de A ou B, ou les copolymères multiséquencés ayant plus de trois séquences différentes de type ABCD.

De manière générale, comme on l'a déjà indiqué, la nature des séquences est choisie de manière à favoriser la séparation des phases entre les séquences, puisque celle-ci prédétermine les propriétés.

La nature des séquences et leur nombre sont choisis par l'homme du métier en fonction des propriétés recherchées dans les limites des conditions spécifiées ci-dessus pour les séquences A et B.

Les copolymères de l'invention sont définis comme étant des copolymères éthyléniques. Cela signifie que les monomères dont sont issus les séquences ou blocs constituant le copolymère sont des monomères à double liaison insaturée carbone-carbone de type éthylénique.

En outre, spécifiquement, le copolymère, selon l'invention, est un copolymère linéaire. Cela signifie que l'invention n'entend pas couvrir les copolymères ayant une structure non linéaire, par exemple ramifiée, en étoile, greffée, ou autre. Le caractère linéaire des copolymères de l'invention est important pour communiquer aux compositions le contenant, les propriétés avantageuses décrites plus haut.

Avantageusement, le copolymère est un polymère filmogène, c'est-à-dire qu'il est apte à lui seul, ou en présence d'agent auxiliaire de filmification, à la température allant de 20°C à 30°C, à former un film continu (vu à l'oeil nu) et adhèrent sur un support kératinique.

Selon l'invention, le copolymère comprend au moins une séquence A susceptible d'être préparée à partir de monomères constitués de 60 à 80% en poids d'un monomère éthylénique à cycle lactame, par exemple un vinyllactame, de formule (I), et de préférence de formule (II).

Comme indiqué plus haut, la teneur (en poids) en monomère éthylénique à cycle lactame est donnée par rapport au poids total des monomères à partir desquels est susceptible d'être préparée la séquence A.

En d'autres termes, la teneur en motifs issus d'un monomère éthylénique à cycle lactame de la séquence A est de 60 à 80% en poids.

Cette teneur en poids est donnée par rapport au poids total de la séquence A.

Le pourcentage de monomère éthylénique à cycle lactame, tel qu'un vinyllactame, de formule (I) ou (II), dans les monomères à partir desquels la séquence A est susceptible d'être préparée est de 60 à 80 % en poids, mieux encore de 65 à 75% en poids par exemple de 70 % en poids.

En d'autres termes, la teneur (en poids) en motifs issus d'un monomère éthylénique à cycle lactame de la séquence A est de 60 à 80% en poids.Et généralement la teneur (en poids) en motifs issus d'un monomère éthylénique à cycle lactame de la séquence A est dans les plages de % en poids définis ci-dessus. Les teneurs et teneurs préférées en poids sont données par rapport au poids total de la séquence A.

La séquence A comprenant de 60 à 80% en poids de monomère éthylénique à cycle lactame, tel qu'un vinyllactame, peut avoir une température de transition vitreuse Tg quelconque, mais elle a généralement une température de transition vitreuse globale de la séquence "haute".

Par "haute", on entend généralement que cette Tg peut être comprise entre 0 et 250°C, de préférence entre 0 à 220°C et de préférence encore entre 5 et 200°C.

La séquence A peut être hydrophile ou non hydrophile, c'est-à-dire hydrophobe.

En effet, suivant le motif hydrophobe contenu dans la séquence A, celle-ci peut rester soluble dans l'eau ou au contraire devenir insoluble.

Une séquence hydrophile peut être définie comme une séquence hydrosoluble ou hydrodispersible. La définition de ces termes est donnée plus loin.

Le monomère éthylénique à cycle lactame peut être choisi parmi les N-vinyllactames et les dérivés de ceux-ci qui peuvent avoir, par exemple, un ou plusieurs substituants alkyle en C₁ à C₆, tels que méthyle, éthyle n-propyle, isopropyle, n-butyle, sec-butyle.

Avantageusement, ledit N-vinyllactame de formule (I) est le pyrrilinoéthylacrylate ou le pyrrilidinoéthylméthacrylate.

Avantageusement, le N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

Les N-vinyllactames préférés sont la N-vinylpyrrolidone, la N-vinylcaprolactame et la vinylimidazolidinone.

Selon l'invention, la séquence A, constituée de 60 à 80% en poids de monomère éthylénique à cycle lactame, tel qu'un N-vinyllactame est un copolymère, les monomères, à partir desquels elle est susceptible d'être préparée, sont donc constituées, outre le monomère éthylénique à cycle lactame, tel qu'un vinyllactame, de formule (I), par un ou plusieurs autres monomères, identiques ou différents, qui sont, selon l'invention, des monomères non hydrophiles ou également nommés hydrophobes.

Ces monomères non hydrophiles sont présents en une quantité, proportion, de 20 à 40% en poids.

Autrement dit, les monomères à partir desquels est susceptible d'être préparée la séquence A, comprennent généralement une proportion de monomère(s) non hydrophile(s), de 20 à 40% en poids, mieux encore de 25 à 35% en poids, par exemple de 30% en poids, par rapport au poids total des monomères à partir desquels est susceptible d'être préparée la séquence A.

En d'autres termes, la teneur (en poids) en motifs issus d'un monomère non hydrophile de la séquence A, est de 20 à 40% en poids. Et généralement la teneur (en poids) en motifs issus d'un monomère non hydrophile se situe dans les plages de % en poids définis ci-dessus. Les teneurs et teneurs préférées sont données par rapport au poids total de la séquence A.

Ce ou ces monomères de la séquence A, autres que le monomère éthylénique à cycle lactame, tel que le vinyllactame, sont des monomères éthyléniques copolymérisables avec le monomère éthylénique à cycle lactame, quel que soit leur coefficient de réactivité.

De préférence, ledit copolymère de la séquence A est un copolymère statistique, mais il peut s'agir aussi d'un copolymère alterné, ou encore d'un copolymère à gradient.

Ce ou ces monomères, autres que le monomère éthylénique à cycle lactame, tel qu'un vinyllactame, à partir duquel(desquels) la séquence A est susceptible d'être préparée, peuvent avoir une température de transition vitreuse de l'homopolymère correspondant "haute" ou "basse", mais ces monomères, toutefois, sont choisis, de préférence, parmi les monomères dont la Tg de l'homopolymère correspondant est « basse », c'est-à-dire, généralement, inférieure ou égale à 50°C, de préférence encore inférieure ou égale à 20°C, et mieux inférieure ou égale à 0°C.

De préférence ces monomères ont, en outre, une Tg de l'homopolymère correspondant qui est supérieure ou égale à -150°C.

La séquence A, comprenant de 60 à 80% en poids de monomère éthylénique à cycle lactame, par exemple en vinyllactame, de formule (I) ou (II), représente généralement de 1 à 99 % en poids du copolymère de l'invention, de préférence de 10 à 95 %, de préférence encore de 20 à 90 %, en poids du poids total du copolymère final.

La ou les séquences B autres que la séquence A est(sont) susceptible(s) d'être préparée(s) à partir d'un ou de plusieurs monomères éthyléniques choisis généralement parmi : les monomères allyliques, les acrylates, les méthacrylates, les acrylamides, les méthacrylamides, les monomères vinyliques, et leurs mélanges, et éventuellement des monomères éthyléniques à cycle lactame, tel qu'un vinyllactame, de formule (I) ou (II), la proportion en poids desdits monomères (I) ou (II) étant minoritaire, inférieure à 50% en poids, de préférence inférieure ou égale à 45 % en poids et, de préférence encore, inférieure ou égale à 40 % en poids, mieux inférieure ou égale à 30 % en poids.

La ou les séquences B peut(peuvent) être hydrophobe(s) ou hydrophile(s), de préférence, une ou plusieurs parmi les séquences B, autres que la séquence A, sont hydrophiles.

De préférence, la séquence B a une température de transition vitreuse Tg, basse, à savoir, de préférence, inférieure ou égale à 50°C, de préférence encore inférieure ou égale à 20°C, mieux inférieure ou égale à 0°C.

De préférence, en outre, la séquence B a une Tg qui est supérieure ou égale à -150°C.

Avantageusement, la masse moléculaire moyenne en nombre de chaque bloc ou séquence, qu'il s'agisse de la séquence A ou de la séquence B, est comprise entre 2000 et 1 000 000, de préférence encore comprise entre 2 000 et 800 000, et mieux comprise entre 2000 et 500 000.

La masse moléculaire moyenne en nombre du copolymère global (par exemple A-b-B) est généralement de 4 000 à 1 000 000, de préférence de 4 000 à 800 000, de préférence encore de 4 000 à 500 000.

Avantageusement, la proportion de la séquence B est comprise entre 1 et 99 % en poids du copolymère, de préférence entre 5 et 90 % et, de préférence encore de 10 à 80 % en poids du poids total du copolymère final.

On va maintenant décrire, de manière plus précise, le copolymère selon l'invention.

La température de transition vitreuse Tg étant un paramètre important pour définir les séquences du copolymère de l'invention, notamment la séquence B du copolymère de l'invention et, par voie de conséquence, le copolymère de l'invention, il est important d'indiquer que les températures de transition vitreuse des séquences des copolymères utilisés dans la présente invention sont mesurées par analyse enthalpique différentielle (DSC, « Differential Scanning Calorimetry », en anglais) pour le polymère sec, à une vitesse de chauffe de 10°C/minute.

Chaque séquence B du copolymère, selon l'invention, est issue d'un type de monomère ou de plusieurs types de monomères différents.

Cela signifie que chaque séquence B peut être constituée d'un homopolymère ou d'un copolymère ; ce copolymère constituant la séquence pouvant être à son tour statistique, alterné, ou encore à gradient.

Chaque séquence A du copolymère selon l'invention est constitué par un copolymère alterné, statistique ou à gradient ; ce copolymère comprenant, bien sûr, une proportion de 60 à 80% en poids de monomère éthylénique à cycle lactame tel qu'un vinyllactame.

Nous allons décrire maintenant en détail chacune des séquences A et B constituant le copolymère selon l'invention.

Selon l'invention, la séquence A, comprenant de 60 à 80% en poids en monomère éthylénique à cycle lactame, tel qu'un vinyllactame, est constituée à savoir de 20 à 40% en poids, par au moins un monomère non hydrophile. Par monomère non hydrophile, on entend que l'homopolymère préparé à partir de ce monomère n'est pas hydrosoluble ou hydrodispersible.

L'homopolymère est hydrosoluble, s'il est soluble dans l'eau, à raison d'au moins 5 % en poids, à 25°C.

L'homopolymère est hydrodispersible, s'il forme à une concentration de 5 %, à 25°C, une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Le ou les monomères non hydrophiles autres que la vinyllactame, à partir desquels est préparé la séquence A, sont, de préférence, choisis parmi les monomères dont la température de transition vitreuse de l'homopolymère correspondant est basse, de préférence encore inférieure ou égale à 50°C de préférence encore inférieure ou égale à 20°C, mieux inférieure ou égale à 0°C.

En outre, le ou lesdits monomères non hydrophiles ont de préférence une Tg supérieure ou égale à -150°C.

Le ou les monomère(s) non hydrophile(s), de la séquence A, peuvent être choisis de préférence parmi les monomères suivants :
- Les hydrocarbures éthyléniques de 2 à 10 C, tels que l'éthylène, l'isoprène, ou le butadiène ;
- Les acrylates de formule CH₂ = CHCOOR₃ ;
- Les méthacrylates de formule : 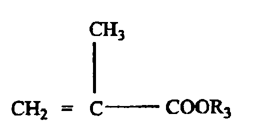 dans lesquelles R₃ représente :
   - un groupe alkyle linéaire, ou ramifié, de 1 à 18 atomes de carbone, dans lequel se trouve(nt) éventuellement intercalé(s) un ou plusieurs hétéroatomes choisis parmi 0, N, S et P,
   ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle,
   - un groupe cycloalkyle en C₃ à C₁₂, tel que le groupe isobornyle,
   - un groupe aryle en C₃ à C₂₀ tel que le groupe phényle,
   - un groupe aralkyle en C₄ à C₃₀ (groupe alkyle en C₁ à C₈) tel que 2- phényl-éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅) , où R₄ et R₅ identiques ou différents représentent un groupe alkyle en C₁ à C₆, ou un groupe phényle,
   - des exemples de groupes R₃ sont les groupes méthyle, éthyle, propyle, isobutyle, n-butyle, tertiobutyle, héxyle, éthylhéxyle, octyle, lauryle, isooctyle, isodécyle, dodécyle, cyclohexyle, t-butylcyclohexyle, t-butylbenzyle, isobornyle, phényle, furfurylméthyle, tétrahydrofurfurylmethyle, éthyl-2-perfluorohexyle,
   µ un autre exemple de groupe R₃ pour les acrylates sont les groupes R₃ = -(OC₂H₄)ₘ-OR'', avec m = 5 à 150 et, R" = H ou alkyle de C₆ à C₃₀, par exemple -POE-lauryle ;
- Les (méth)acrylamides de formule : 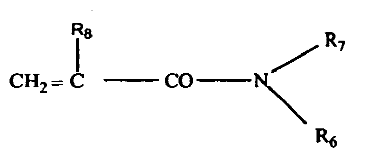 où R₈ désigne H ou méthyle,
   et R₇ et R₆ identiques ou différents représentent chacun un atome d'hydrogène ou un groupe alkyle de 1 à 18 atomes de carbone linéaire ou ramifié, dans lequel se trouve(nt) , éventuellement intercalé(s) un ou plusieurs hétéroatomes choisis parmi 0, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents, représentent un groupe alkyle en C₁ à C₆, ou un groupe phényle,
   des exemples de ces groupes sont les groupes méthyle, éthyle, n-butyle, t-butyle, isopropyle, isohexyle, isooctyle, isononyle,
   - un groupe cycloalkyle en C₃ à C₁₂, tel que le groupe isobornyle,
   - un groupe aryle en C₃ à C₂₀ tel que phényle,
   - un groupe aralkyle en C₄ à C₃₀ (groupe alkyle en C₁ à C₈) tel que 2- phényl éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F) et les groupes Si(R₄R₅), où R₄ et R₅ identiques ou différents représentent, un groupe alkyle en C₁ à C₆, ou un groupe phényle.
   Des exemples de monomères (méth)acrylamide sont le N-éthyl(méth)acrylamide, le N-butylacrylamide, le N-t-butylacrylamide, le N-isopropylacrylamide, le N,N-dibutylacrylamide, le N-octylacrylamide, le N-dodécylacrylamide, et l'undécylacrylamide ;
- Les composés allyliques de formule :

   CH₂ = CH-CH₂-R₉ ou CH₂ = C(CH₃)-CH₂-R₉ ;
- Les composés vinyliques de formule :

   CH₂ = CH-R₉,

   où R₉ est un groupe :
   - OR₁₀, où R₁₀ représente un groupe phényle ou un groupe alkyle en C₁ à C₁₂ (le monomère est un éther de vinyle ou d'allyle),
   - OCOR₁₁, où R₁₁ représente :
      - un groupe alkyle de 2 à 12 C linéaire ou ramifié (le monomère est un ester de vinyle ou d'allyle) ;
      - un groupe cycloalkyle en C₃ à C₁₂ tel que isobornyle, cyclohexyle,
      - un groupe aryle en C₃ à C₂₀ tel que phényle,
      - un groupe aralkyle en C₄ à C₃₀ (groupe alkyle en C₁ à C₈) tel que 2-phényléthyle, benzyle,
      - un groupe hétérocyclique de 4 à 12 chaînons contenant un ou plusieurs hétéroatomes choisis parmi O, N, et S, le cycle étant aromatique ou non,
      - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅) où R₄ et R₅ identiques ou différents représentent un groupe alkyle en C₁ à C₆, ou un groupe phényle.

Des exemples de monomères vinyliques sont le vinylcyclohexane, et le styrène.

Des exemples d'esters de vinyle sont : l'acétate de vinyle, le propionate de vinyle, le butyrate de vinyle, l'éthylhexanoate de vinyle, le néononanoate de vinyle, et le néododécanoate de vinyle.

Parmi les éthers de vinyle on trouve par exemple le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

Les monomères, particulièrement préférés, sont choisis parmi ceux dont la température de transition vitreuse Tg de l'homopolymère correspondant est inférieure ou égale à 50°C tels que : l'acrylate de méthyle (Tg=10°C), l'acrylate d'éthyle (Tg=-24°C), l'acrylate de n-butyle (Tg=-54°C), l'acrylate de t-butyle (Tg=43°C) l'acrylate de 2 d'éthylhexyle (Tg=-50°C), l'acrylate d'isobutyle (-24°C), le méthacrylate de butyle (Tg=20°C), le méthacrylate de n-hexyle, le méthacrylate de POE (n=8 à 10) (Tg=-55°C), l'acétate de vinyle (Tg=23°C) ; et d'autres monomères, tels que le méthacrylate de méthyle (Tg=105°C), le méthacrylate d'éthyle, le méthacrylate d'isobutyle, l'acrylate de furfuryle, l'acrylate d'isobornyle, l'acrylate de tert-butylcyclohexyle, le styrène et le vinylcyclohexane.

Les monomères non hydrophiles préférés entre tous pour constituer la séquence A sont choisis parmi l'acrylate de t-butyle, l'acrylate de méthyle, l'acrylate d'éthyle et l'acrylate de butyle.

Le copolymère selon l'invention comprend également une ou plusieurs autres séquences B qui est (sont) susceptible(s) d'être préparée(s) à partir de monomères ne comportant pas de monomère éthylénique à cycle lactame, tel qu'un vinyllactame de formule (I) ou comportant une proportion minoritaire de celui-ci.

Il n'existe aucune limitation quant à la nature des monomères qui sont susceptibles de donner la séquence B, à l'exception, bien sûr, de la limitation quant à la quantité de monomère éthylénique à cycle lactame, tel qu'un vinyllactame susceptible d'être utilisée pour la préparation de cette séquence B.

Ces monomères peuvent être hydrophobes, non hydrophiles, mais ils peuvent aussi être hydrophiles. Les termes « hydrophile » et a contrario « non hydrophile » ont déjà été définis plus haut.

Le ou les monomère(s), autres que le monomère éthylénique à cycle lactame, tel qu'un vinyllactame, minoritaire, à partir duquel ou desquels la séquence ou les séquences B est (sont) susceptible(s) d'être préparée(s) peut (peuvent ) être choisi(s) parmi une grande variété de monomère(s), les Tg de ces monomères peuvent être quelconques, et ces monomères peuvent en outre être hydrophiles ou hydrophobes.

De façon préférée, la séquence B a une température de transition vitreuse Tg basse, à savoir de préférence inférieure ou égale à 50°C, de préférence encore inférieure à 20°C, mieux inférieure à 0°C.

Pour cela le ou les monomères, autres que le monomère éthylénique à cycle lactame (tel qu'un vinyllactame) minoritaire à partir duquel ou desquels est susceptible d'être préparée la séquence B, peuvent être de préférence, choisis parmi les monomères dont la température de transition vitreuse Tg de l'homopolymère correspondant est basse, de préférence encore inférieure ou égale à 50°C, mieux inférieure ou égale à 20°C, et encore mieux inférieure ou égale à 0°C, de préférence, en outre, ce ou ces monomères ont, de préférence, une température de transition vitreuse de l'homopolymère correspondant supérieure à -150°C.

Les monomères de la séquence B peuvent également être choisis de façon à ce qu'en combinant, dans le copolymère, des monomères de Tg (de l'homopolymère correspondant) hautes et des monomères de Tg basse, la Tg globale de la séquence soit comprise dans les fourchettes souhaitées. Ainsi, par exemple, en combinant pour préparer la séquence B, un monomère de Tg (de l'homopolymère correspondant) égale à 100°C (par exemple le méthacrylate de méthyle), à raison de 35 % en poids du poids total des monomères de la séquence B, et un monomère de Tg égale à -50°C, par exemple l'acrylate de 2-éthylhexyle, à raison de 65 % en poids, et la séquence résultante aura une Tg de -14°C, calculée en première approximation par la loi de Fox : 1/Tg = Somme (% Wa/Tga) avec Wa = % en poids du monomère a, et Tga = température de transition vitreuse de l'homopolymère constitué à partir du monomère a.

Ces monomères peuvent être choisis parmi les monomères non hydrophiles, déjà cités plus haut, pour la séquence A.

A la différence de la séquence A, les monomères, à partir desquels peut être préparée la séquence B, peuvent aussi être choisis parmi les monomères hydrophiles.

Des exemples de monomères hydrophiles incluent les monomères ioniques tels que les monomères cationiques, les monomères anioniques, et les bétaïnes ; les monomères non ioniques ; et les monomères pouvant être rendus hydrophiles par suite d'une hydrolyse en particulier des monomères de type esters (méth)acryliques rendus hydrosolubles par transformation en l'acide carboxylique correspondant.

Des exemples de monomères cationiques sont :
- la 2-vinylpyridine (Tg : 104°C) ;
- la 4-vinylpyridine (Tg : 142°C) ;
- le (méth)acrylate de diméthylaminoéthyle ;
- le (méth)acrylate de diéthylaminoéthyle ;
- le diméthylaminopropyl(méth)acrylamide ; et
les formes salifiées ou quaternisées de ceux-ci, qu'il s'agisse de sels d'acides minéraux, tels que l'acide sulfurique ou l'acide chlorhydrique, ou de sels d'acides organiques.

Ces acides organiques peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle.

Un exemple d'acide à groupe alkyle est l'acide acétique CH₃COOH.

Un exemple de polyacide est l'acide téréphtalique.

Des exemples d'hydroxyacides sont l'acide citrique et l'acide tartrique.

Des exemples de monomères anioniques sont :
- l'acide acrylique, l'acide méthacrylique, l'acide crotonique, l'anhydride maléique, l'acide itaconique, l'acide fumarique, l'acide maléique ;
- l'acide styrènesulfonique, l'acide 2-acrylamido 2-methylpropanesulfonique, l'acide vinylsulfonique, l'acide vinylbenzoïque, le méthacrylate de sulfopropyle, l'acide vinylphosphorique, et les sels de ceux-ci.

Le neutralisant peut être une base minérale, telle que LiOH, NaOH, KOH, Ca(OH)₂, NH₄OH, une base organique. Il peut s'agir également d'une alkylamine primaire, secondaire, ou tertiaire, telle que la triéthylamine, ou la butylamine. Cette alkylamine primaire, secondaire ou tertiaire peut comporter des azotes et/ou oxygènes et donc comporter par exemple une fonction alcool, il peut s'agir ainsi par exemple l'amino-2-méthyl-2-propanol, la triéthanolamine.

Des exemples de monomères ioniques de type bétaïne sont :
- les carboxybétaïnes ou sulfobétaïnes éthyléniques obtenues, par exemple par quaternisation de monomères à insaturation éthylénique comportant une fonction amine par des sels d'acide carboxylique à halogène mobile, par exemple, le chloroacétate de sodium, ou par des sulfones cycliques, par exemple la propanesulfone.

Des exemples de monomères non-ioniques sont :
- les (méth) acrylates ou (métha) crylamides d'hydroxyalkyle dont le groupe alkyle a de 2 à 4 atomes de C, en particulier le (méth)acrylate d'hydroxyéthyle ou d'hydroxypropyle ;
- les (méth)acrylates ou (métha)crylamides d' alcoxy (C₁₋₄) alkyle (C₁₋₄) tel que méthoxyéthyle, éthoxyéthyle et méthoxypropyle,
- les (méth)acrylates ou (métha)crylamides à groupe -(OC₂H₄)ₘ-OR''', avec m = 5 à 150 et, R''' = H ou alkyle de C₁ à C₄, par exemple -POE- méthoxy ;-POE-OH ;
- les vinyllactames,
- et les (méth)acrylates de polysaccharide comme l'acrylate de saccharose.

Les vinyllactames ne peuvent, toutefois, constituer qu'une proportion minoritaire de la séquence B.

Les monomères pouvant être rendus hydrophiles par suite d'une hydrolyse sont en particulier les monomères de type esters (méth)acryliques hydrolysables en acide tels que les (méth)acrylate d'éthyle, de tertiobutyle, de benzyle.

On procédera à l'hydrolyse, une fois le polymère synthétisé, selon les conditions acides (tels que l'acide sulfurique, acide chlorhydrique ou acide trifluoroacétique) ou basique (en présence d'hydroxydes de métaux alcalinoterreux tels que la soude ou la potasse, d'alcoolates de métaux alcalins tels que le t-butylate de potassium ou d'amines tels que l'ammoniaque). L'hydrolyse a généralement lieu entre 5 et 100°C, de préférence entre 15 et 80°C.

Le polymère sera ensuite purifié par précipitations répétées.

Les monomères hydrophiles préférés sont :
- l'acide acrylique, l'acide méthacrylique, l'acide crotonique, l'anhydride maléique, l'acide vinylbenzoïque, l'acide styrènesulfonqiue, l'acide 2-acrylamido-2-méthylpropanesulfonique, l'acide vinylsulfonique,
- les vinyllactames,
- les (méth)acrylamides, les (méth)acrylamides de N-alkyle en C₁-C₄, comme l'acrylamide d'isobutyle,
- et les (méth)acrylates de polysaccharide comme l'acrylate de saccharose,
- l'acrylate de tertiobutyle ou l'acrylate d'éthyle suivi de l'hydrolyse.

Il est à noter que même si le copolymère comprend une séquence hydrophile, le copolymère global n'est pas forcément hydrophile.

Les copolymères éthyléniques, séquencés, linéaires selon l'invention sont choisis parmi :
- les copolymères biséquencés (AB) ;
- les copolymères triséquencés (ABA, BAB, ABC, ACB), avec C différent de A ou B,
- les copolymères multiséquencés ayant plus de trois séquences : (AB)ₙ, (ABA)ₙ, (BAB)ₙ, (ABC)ₙ, (ACB)ₙ, avec C différent de A ou B ou les copolymères multiséquencés ayant plus de trois séquences différentes, de type ABCD.

Les copolymères selon l'invention peuvent être préparés par polymérisation par voie anionique.

De préférence, toutefois, les copolymères, selon l'invention, sont dans un premier mode obtenus par polymérisation radicalaire contrôlée et, de manière particulièrement préférée, les copolymères selon l'invention peuvent être obtenus par une polymérisation par « ATRP » particulière qui est la technique dite « Reverse ATRP », ou par la technique « RAFT », mais les polymères selon l'invention peuvent aussi, selon un second mode, être obtenus par polymérisation radicalaire classique.

### Premier mode

Les copolymères blocs ou séquences selon l'invention sont de préférence obtenus par polymérisation radicalaire contrôlée, décrite notamment dans "New Method of Polymer Synthesis", Blackie Académie & Professional, Londres, 1995, volume 2, page 1.

La polymérisation radicalaire controlée permet de réduire les réactions de désactivation de l'espèce radicalaire en croissance, en particulier l'étape de terminaison, réactions qui, dans la polymérisation radicalaire classique, interrompent la croissance de la chaîne polymérique de façon irréversible et sans contrôle.

Afin de diminuer la probabilité des réactions de terminaison, il a été proposé de bloquer de façon transitoire et réversible, l'espèce radicalaire en croissance, en formant des espèces actives dites "dormantes" sous forme de liaison de faible énergie de dissociation.

Ainsi, la polymérisation peut être effectuée selon la technique de transfert d'atome (Atom Transfer Radical Polymerization ou « ATRP », en anglais), ou par réaction avec un nitroxyde, ou bien encore selon la technique de "*reversible addition-fragmentation chain transfer"* (« RAFT ») ou enfin par la technique de « ATRP » inverse, dite « reverse ATRP ».

La technique de polymérisation radicalaire par transfert d'atomes, aussi connue sous l'abréviation ATRP, consiste à bloquer l'espèce radicalaire en croissance sous forme de liaison de type C-halogénure (en présence de complexe métal/ligand). Ce type de polymérisation se traduit par un contrôle de la masse des polymères formés et par un faible indice de dispersité des masses.

D'une manière générale, la polymérisation radicalaire par transfert d'atomes s'effectue par polymérisation d'un ou de plusieurs monomères polymérisables par voie radicalaire, en présence :
- d'un amorceur ayant au moins un atome d'halogène transférable ;
- d'un composé halogéné comprenant un métal de transition susceptible de participer à une étape de réduction avec l'amorceur et une chaîne polymérique "dormante", celui-ci sera dénommé « agent de transfert de chaîne » ; et
- d'un ligand pouvant être choisi parmi les composés comprenant un atome d'azote (N), d'oxygène (O), de phosphore (P) ou de soufre (S), susceptibles de se coordonner par une liaison σ audit composé comprenant un métal de transition, la formation de liaisons directes entre ledit composé comprenant un métal de transition et le polymère en formation étant évitées.

L'atome d'halogène est de préférence un atome de chlore ou de brome.

Ce procédé est en particulier décrit dans la demande WO 97/18247 et dans l'article de Matyjasezwski *et al*. publié dans *JACS,* 117, page 5614 (1995).

La technique de polymérisation radicalaire par réaction avec un nitroxyde consiste à bloquer l'espèce radicalaire en croissance sous forme de liaison de type C-O-NRₐR_{b}, Rₐ et R_{b} peuvent être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle. Cette technique de polymérisation est notamment décrite dans les articles « Living free radical polymerization : a unique technique for prepration of controlled macromolecular architectures » CJ Hawker ; Chem. Res., 1997, 30, 373-382 et "Macromolecular engineering via living free radical polymerizations" publié dans Macromol. Chem. Phys. 1998, vol. 199, pages 923 - 935, ou bien encore dans la demande WO-A-99/03894.

La technique de polymérisation RAFT *(reversible addition-fragmentation chain transfer)* consiste à bloquer l'espèce radicalaire en croissance sous forme de liaison de type C-S. On utilise pour cela des composés dithio comme les dithioesters (-C(S)S-), tels que les dithiobenzoates, les dithiocarbamates (-NC(S)S-) ou les dithiocarbonates (-OC(S)S-) (xanthates). Ces composés permettent de contrôler la croissance de la chaîne d'une large gamme de monomères. Toutefois, les dithioesters inhibent la polymérisation des esters vinyliques, tandis que les dithiocarbamates sont très faiblement actifs vis-à-vis des méthacrylates, ce qui limite dans une certaine mesure l'application de ces composés. Cette technique est notamment décrite dans la demande WO-A-98/58974 de RHODIA et dans l'article "A more versatile route to block copolymers and other polymers of complexe architecture by living radical polymerization : the RAFT process", publié dans Macromolecules, 1999, volume 32, pages 2 071-2 074. La demande WO-A-98/58974 déjà citée et la demande WO-A-99/31144 de CSIRO ont trait à l'utilisation de dithiocarbamates en tant que réactifs « RAFT ». En utilisant ces dithiocarbamates divers monomères sont polymérisés dont l'acétate de vinyle.

Les avantages principaux de la technique « RAFT » sur la technique « ATRP » sont qu'elle ne nécessite pas de catalyseur métallique et également que des amorceurs de radicaux classiques peuvent être utilisés pour amorcer la réaction.

Dans le cas de la polymérisation radicalaire contrôlée, par exemple de la N-vinylpyrrolidone (NVD), des agents de transfert de chaîne préférés utilisés dans le cas de la polymérisation « RAFT » sont les dérivés de diphényldithiocarbamate.

En faisant varier le rapport de la concentration en monomère sur la concentration en agent de transfert de chaîne, la masse moléculaire du polymère peut être modifiée.

La polymérisation se déroule en plusieurs étapes selon le schéma général :
a - Dans une première étape, on effectue la polymérisation du premier monomère ou mélange de monomères pour former un macroamorceur,
b - Les polymères purifiés par précipitation sont séchés sous vide,
c - Ensuite, dans la deuxième étape, on réalise la polymérisation de la seconde séquence constituée par un monomère ou un mélange de monomères à l'extrémité du macroamorceur (formé à l'étape a)).

Les étapes b et c sont répétées autant de fois que nécessaire suivant le nombre de séquences.

Un procédé particulièrement privilégié est le procédé de polymérisation dit « ATRP » inverse (« Reverse ATRP », en anglais) à ne pas confondre avec le procédé de ATRP classique décrit plus haut.

Dans ce procédé de « Reverse ATRP », l'amorçage est réalisé de façon classique par un amorceur capable de donner des radicaux, tels que par exemple l'azobisisobutyronitrile (AIBN) ou un peroxyde (et non plus à l'aide d'un amorceur spécifique).

La présence d'halogénures de métal, tel que CuBr₂ et d'un ligand permet le contrôle de la polymérisation par « capture » réversible des radicaux formés.

Les ligands préférés sont des molécules à base d'amine en particulier la tris (diméthylaminoéthyl) amine (Me₆TREN). Un bon contrôle de la polymérisation est observé en particulier avec le système AIBN/CuBr₂/Me₆TREN.

L'autre procédé privilégié est le procédé de polymérisation dit "RAFT".

### Deuxième mode

Les polymères blocs ou séquences selon l'invention peuvent également être obtenus en utilisant la technique de polymérisation radicalaire classique en effectuant la coulée des monomères de façon séquencée. Dans ce cas, seul le contrôle de la nature des séquences est possible (pas de contrôle des masses).

Il s'agit de polymériser dans un premier temps un monomère M1 dans un réacteur de polymérisation; de suivre, par cinétique, sa consommation dans le temps puis quand M1 est consommé à environ 95% alors d'introduire un nouveau monomère M2 dans le réacteur de polymérisation.

On obtient ainsi un polymère de structure bloc de type M1-M2.

De manière plus précise, un premier procédé de préparation d'un copolymère tel que décrit ci-dessus comprenant au moins une séquence A et au moins une séquence B et éventuellement une ou plusieurs autres séquences différentes des séquences A et B, dans lequel la polymérisation est réalisée par la technique dite de polymérisation radicalaire à transfert d'atomes inverse (« Reverse ATRP ») comprend les étapes successives suivantes :
a) on polymérise le monomère ou les monomères à partir desquels est préparée la séquence A en présence d'un agent de transfert de chaîne, tel qu'un halogénure de métal de transition, d'un amorceur de radicaux, et d'un ligand, et en présence ou non d'un solvant, moyennant quoi, on obtient un macroamorceur ou précurseur fonctionnel capable d'amorcer la polymérisation car comportant à ses extrémités la fonction agent de transfert de chaîne ;
b) on polymérise le monomère ou les monomères à partir desquels est préparée la séquence B à l'extrémité dudit macroamorceur ou précurseur en présence d'un agent de transfert de chaîne tel qu'un halogénure de métal de transition, d'un amorceur et d'un ligand, et en présence ou non d'un solvant, moyennant quoi on obtient un copolymère biséquencé de structure A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

De façon à obtenir des copolymères triséquencés symétriques, il est possible d'utiliser des amorceurs difonctionnels.

Avantageusement, les « agents de transfert de chaîne » sont choisis parmi les halogénures de métaux à l'état d'oxydation le plus élevé, de préférence parmi CuBr₂, CuCl₂, FeCl₂P(phényl)₃, FeCl₃, et RuCl₂P(phényl)₃.

Avantageusement, les amorceurs de polymérisation radicalaire sont choisis parmi :
- les composés azoïques, tels que le 2,2'-azobisisobutyronitrile (AIBN), le 2,2'-azobis(2-butanenitrile), le 4,4'-azobis(4-acide pentanoïque), le 1,1'-azobis(cyclohexanecarbonitrile), le 2-(t-butylazo)-2-cyanopropane, le 2,2'-azobis[2-méthyl-N-(1,1)-bis(hydrox yméthyl)-2-hydroxyéthyl]propionamide, le 2,2'-azobis(2-méthyl-N-hydroxyéthyl) propionamide, le dichlorure de 2,2'-azobis(N,N'-diméthylèneisobutyrami dine), le dichlorure de 2,2'-azobis(2-amidinopropane), le 2,2'-azobis(N,N'-diméthylèneisobutyrami de), le 2,2'-azobis(2-méthyl-N-[1,1-bis(hydroxy méthyl)-2-hydroxyéthyl]propionamide), le 2,2'-azobis(2-méthyl-N-[1,1-bis (hydroxyméthyl)éthyl] propionamide), le 2,2'-azobis(2-méthyl-N-(2-hydroxyéthyl) propionamide), le 2,2'-azobis(isobutyramide)dihydrate ;
- les peroxydes d'hydrogène, tels que l'hydropéroxyde de butyle tertiaire, l'hydropéroxyde de cumène, le t-butyl-peroxyacétate, le t-butylperoxybenzoate, le t-butylperoxyoctoate, le t-butylperoxynéodécanoate, le t-butylpéroxyisobutarate, le peroxyde de lauroyle, le t-amylperoxypivalate,le t-butylperoxypivalate, le peroxyde de dicumyle, le peroxyde de benzoyle ;
- les persulfates alcalins, tels que le persulfate de potassium, le persulfate d'ammonium ;
- les systèmes redox comportant des combinaisons, telles que :
   - les mélanges de peroxyde d'hydrogène, d'alkyle, peresters, percarbonates et similaires et de n'importe lequel des sels de fer, de sels titaneux, formaldéhyde sulfoxylate de zinc ou formaldéhyde sulfoxylate de sodium, et des sucres réducteurs,
   - les persulfates, perborate ou perchlorate de métaux alcalins ou d'ammonium en association avec un bisulfite de métal alcalin, tel que le métabisulfite de sodium, et des sucres réducteurs,
   - les persulfates de métal alcalin en association avec un acide arylphosphinique, tel que l'acide benzène phosphonique et autres similaires, et des sucres réducteurs.

Avantageusement, les ligands sont choisis parmi les ligands tétradentate, tels que le 1,1,4,7,10,10 hémaméthyltriéthylènetétramine (HMTETA), le 1,4,8,11 tétraazacyclotétradécane (cyclame), le 1,4,8,11 tétraméthyl-1,4,8,11-tétraazacyclotétradécane (Me₄ cyclame), et la tris(diméthylaminoéthyl)amine (Me₆TREN) ; les ligands hexadentate, tels que la tétra 2-pyridyl pyrazine (TPPY), la N,N,N',N' tétrabis(2-pyridyl méthyl)éthylène diamine (TPMEDA) ; et la triphénylphosphine (TPP).

Avantageusement, les solvants sont choisis parmi le dioxane, le tétrahydrofurane (THF), la N-méthylpyrrolidone, l'eau et leurs mélanges.

Les catalyseurs, amorceurs, ligands, solvants peuvent être identiques ou différents dans l'étape a) et l'étape b), mais de préférence, ils sont identiques. De préférence encore dans la première et la deuxième étape a) et b), l' « agent de transfert de chaîne » est CuBr₂, l'amorceur est AIBN, le ligand est Me₆TREN, et le solvant est le dioxane.

Ce premier procédé peut permettre de préparer l'un quelconque parmi les copolymères de l'invention, mais il s'applique particulièrement à la préparation d'un copolymère dans lequel la séquence A est un copolymère issu d'un monomère "majoritaire" choisi parmi la N-vinylpyrrolidone et la N-vinylcaprolactame et d'un monomère "minoritaire" qui est l'acrylate de t-butyle, ou l'acrylate de butyle par exemple et la séquence B est par exemple un homopolymère issu d'un monomère choisi parmi l'acrylate de méthyle, l'acrylate de t-butyle et le méthacrylate de méthyle.

Un second procédé de préparation d'un copolymère tel que décrit ci-dessus comprenant au moins une séquence A et au moins une séquence B et éventuellement une ou plusieurs autres séquences différentes des séquences A et B, dans lequel la polymérisation est réalisée par la technique dite de polymérisation RAFT « Réversible Addition-Fragmentation Chain Transfert » comprend les deux étapes successives suivantes :
a) on polymérise le ou les monomères à partir desquels est préparée la séquence A en présence d'un agent de transfert de chaîne, et d'un amorceur, dans un solvant ou non, moyennant quoi on obtient un macroamorceur ou précurseur comportant à ses extrémités la fonction agent de transfert de chaîne ;
b) on polymérise le ou les monomères à partir desquels est préparée la séquence B à l'extrémité dudit macroamorceur ou précurseur, en présence d'un amorceur, dans un solvant ou non, moyennant quoi on obtient un copolymère A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

Avantageusement, les agents de transfert de chaîne sont choisis parmi les dithioesters (-C(S)S-) tels que les dithiobenzoates, les dithiocarbamates (-NC(S)S-) ou les dithiocarbonates (-OC(S)S-)(xanthates).

De préférence, les agents de transfert de chaîne sont choisis parmi le diphényl dithiocarbamate de malonate de diéthyle (DPCM), le diphényldithiocarbamate d'acétate de fluoroéthyle (DPFEM) et le xanthate de formule C₂H₅OC(S) SCH (CH₃) COOCH₃.

Avantageusement, les amorceurs sont choisis parmi les composés déjà cités ci-dessus pour le premier procédé.

Avantageusement, les solvants sont choisis parmi le dioxane, le tétrahydrofuranne, la N-méthylpyrrolidone, l'eau, et leurs mélanges.

Les amorceurs, agents de transfert de chaîne et solvants peuvent être identiques ou différents dans l'étape a) et l'étape b). De préférence, ils sont identiques.

De préférence encore dans la première et la deuxième étapes a) et b), l'amorceur est AIBN, l'agent de transfert de chaîne est le diphényl dithiocarbamate de malonate de diéthyle (DPCM), le diphényldithiocarbamate d'acétate de fluoroéthyle (DPFEM) ou le xanthate de formule C₂H₅OC(S)SCH(CH₃)COOCH₃ suivant la nature des monomères à polymériser, et le solvant est le dioxane.

Ce second procédé peut permettre de préparer l'un quelconque parmi les copolymères de l'invention, mais il s'applique particulièrement à la préparation d'un copolymère dans lequel la séquence A est un comopolymère issu d'un monomère "majoritaire" choisi parmi la N-vinylpyrrolidone et la N-vinylcaprolactame et d'un monomère "minoritaire" qui est l'acrylate de t-butyle ou l'acrylate de butyle et la séquence B est un homopolymère issu d'un monomère choisi parmi l'acrylate de méthyle, l'acrylate de t-butyle et le méthacrylate de méthyle

L'invention concerne également les compositions cosmétiques ou pharmaceutiques comprenant le copolymère de structure spécifique, tel qu'il a été décrit ci-dessus.

Généralement, ces compositions contiennent de 0,1 à 60 % en poids, de préférence de 0,5 à 50 % en poids, et de préférence encore de 1 à 40 % en poids du copolymère selon l'invention.

Ces compositions, notamment cosmétiques, selon l'invention, comprennent, outre ledit copolymère, un milieu physiologiquement acceptable, c'est-à-dire un milieu compatible avec les matières kératiniques, comme la peau, les cheveux, les cils, les sourcils et les ongles.

De manière générale, il faut considérer que l'ensemble de la composition est physiologiquement acceptable.

Ledit milieu, physiologiquement acceptable, comprend généralement un ou plusieurs solvant(s) approprié(s), physiologiquement acceptable(s), dans lequel le copolymère, selon l'invention, se trouve sous forme dissoute ou dispersée.

La composition, par exemple cosmétique, peut ainsi comprendre, en tant que solvant(s) approprié(s) formant une phase hydrophile, un ou plusieurs solvants choisis parmi l'eau et les mélanges d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols. La phase hydrophile peut, en outre, contenir des éthers en C₂.

L'eau ou le mélange d'eau et de solvants organiques hydrophiles peut être présent dans la composition selon l'invention en une teneur allant de 0 % à 90 % (notamment 0,1 % à 90 %) en poids, par rapport au poids total de la composition, et de préférence de 0 % à 60 % en poids (notamment 0,1 % à 60 % en poids).

Le milieu physiologiquement acceptable de la composition peut comprendre, en outre, une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu physiologiquement acceptable de la composition selon l'invention peut également comprendre un ou plusieurs solvants organiques, cosmétiquement et/ou pharmaceutiquement acceptables (tolérance, toxicologie et toucher acceptables). Ces solvants peuvent être présents généralement en une teneur allant de 0 à 90 %, de préférence de 0,1 à 90 %, de préférence encore de 10 à 90% en poids, par rapport au poids total de la composition, et mieux de 30 à 90 %.

Comme solvants utilisables dans la composition de l'invention, on peut citer les esters de l'acide acétique comme l'acétate de méthyle, d'éthyle, de butyle, d'amyle, de méthoxy-2-éthyle, l'acétate d'isopropyle; les cétones comme la méthyléthylcétone, la méthylisobutylcétone ; les hydrocarbures comme le toluène, le xylène, l'hexane, l'heptane ; les aldéhydes ayant de 5 à 10 atomes de carbone ; les éthers ayant au moins 3 atomes de carbones ; et leurs mélanges.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.

Le copolymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu physiologiquement acceptable de la composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants hydrosolubles sont par exemple le jus de betterave, le bleu de méthylène.

Le milieu physiologiquement acceptable de la composition, par exemple cosmétique, selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls@ de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Le milieu physiologiquement acceptable de la composition selon l'invention peut également contenir des ingrédients couramment utilisés en cosmétique et/ou en pharmacie, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les polymères hydrosolubles, liposolubles ou en dispersion dans l'eau ou dans une phase gazeuse, filmogènes ou non, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition, par exemple cosmétique, selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 s⁻¹, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara ou laque pour cheveux).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray.

Le copolymère selon l'invention lorsqu'il est utilisé dans les produits capillaires tels que les produits pour le maintien de la coiffure ou la mise en forme des cheveux permet d'éviter le poudrage " flaking ".

Les solutions peuvent être conditionnées sous diverses formes : sels, lotions, par exemple, et notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

On a vu plus haut que le polymère selon l'invention était particulièrement adapté à une mise en oeuvre dans de telles conditions car il présentait une grande compatibilité avec les gaz propulseurs utilisés notamment dans les récipients aérosols. Le ou les agents propulseurs peuvent être choisis parmi le diméthyléther, les alcanes en C₃₋₅, tels que le propane, le n-butane et l'isobutane ; le 1,1-difluoroéthane, les mélanges de diméthyléther et d'alcanes en C₃₋₅, et les mélanges de 1,1-difluoroéthane et de diméthyléther et/ou d'alcanes en C₃₋₅.

L'invention va maintenant être décrite, en référence aux exemples suivants, donnés à titre illustratif et non limitatif.

### Exemples

Dans les exemples suivants, on prépare des polymères selon l'invention, par la technique de polymérisation radicalaire par transfert d'atomes inverse, aussi connue sous l'abréviation « Reverse ATRP », ou par la technique de polymérisation « RAFT » (Reverse Addition - Fragmentation Chain Transfer).

De manière générale, la formation de blocs dans le cas du procédé « ATRP » inverse et du procédé « RAFT » se déroule en plusieurs étapes.

Ainsi, dans le cas d'un copolymère dibloc, on procédera par exemple, de la manière suivante :
- a) La première étape consiste en une polymérisation du premier monomère pour former le macroamorceur ou précurseur.
- b) La seconde étape consiste en la polymérisation du second monomère à l'extrémité du macroamorceur, pour former le dibloc.

Entre les deux étapes, une étape de purification peut être nécessaire, en particulier dans le cas de la polymérisation dite « Reverse ATRP ».

On décrira tout d'abord le mode opératoire général utilisé pour la réalisation des exemples, pour chacun des procédés : procédé 1 (polymérisation « Reverse ATRP ») et procédé 2 (polymérisation « RAFT »).

### 1. Procédé 1 : « ATRP » inverse

On réalise le procédé de « Reverse ATRP », de préférence avec la tris(diméthylaminoéthyl)amine (TREN Me₆)en tant que ligand, en utilisant CuBr₂ et en mettant en oeuvre AIBN en tant qu'amorceur.

### 1. 1. Matières premières

- Le monomère éthylénique à cycle lactame, tel que la N-vinylpyrrolidone (VP), acquis auprès de la Société ALDRICH®, est distillé sous vide et stocké sous azote à 0°C avant utilisation.
- Les autres monomères, tels que acrylate de tertiobutyle, méthacrylate de méthyle, acquis auprès de la Société ALDRICH® sont séchés sur hydrure de calcium et distillés sous vide.
- L'amorceur azobisisobutyronitrile (AIBN) est recristallisé dans le méthanol.
- Le CuBr₂ (99,9 %), le CuBr, la poudre de cuivre (99 %) sont acquis auprès de la Société ALDRICH® et utilisés tels qu'ils sont fournis ;
- La tris(diméthylaminoéthyl)amine (TREN Me₆) est synthétisée selon les procédures décrites dans la littérature, par exemple dans le document de Matyjaszewski ACS Symp Ser 2000 ; 760 ; 207.
- Tous les solvants : THF, dioxane, et DMF sont séchés par distillation sur CaH₂ avant leur utilisation.

### 1. 2. Polymérisation

La procédure générale est la suivante :

### 1. 2. 1. Première étape

### Formation du macroamorceur ou précurseur

CuBr₂ et Me₆ TREN sont mis dans un ballon et on leur additionne du dioxane.

La solution est agitée durant 30 min. à 25°C.

Le ou les monomère(s) (suivant la séquence préparée en premier et formant le macroamorceur) est(sont) additionné(s) puis l'amorceur AIBN.

Le système est soumis à trois cycles de séchage par vide/argon. La solution est ensuite chauffée dans un bain thermostaté.

La viscosité augmente. Après réaction pendant la durée voulue, le système est refroidi à température ambiante.

Le polymère macroamorceur est séparé par précipitation : il est dilué dans le chloroforme puis précipité dans le diéthyléther, cette opération est répétée deux fois. Le complexe de cuivre est séparé du polymère dissous dans le chloroforme par passage sur colonne neutre. La solution de polymère est alors évaporée.

### 1. 2. 2. Deuxième étape

### Polymérisation de la seconde séquence à l'extrémité du macroamorceur

Le macroamorceur obtenu dans la première étape est additionné à une solution de CuBr et de Me₆TREN dans le dioxane sous atmosphère inerte d'azote.

La quantité requise de manomère(s) constituant la seconde séquence est additionnée. La solution est chauffée. Après le temps désiré, le système est refroidi à température ambiante.

Le polymère est séparé par précipitation : il est dilué dans le chloroforme puis précipité dans le diéthyl éther, cette opération est répétée deux fois. Le complexe de cuivre est séparé du polymère dissous dans le chloroforme par passage sur colonne neutre. La solution de polymère est évaporée.

Le polymère est séché.

Le polymère isolé est généralement une poudre blanche.

### 2. Procédé 2 : « RAFT »

On réalise la polymérisation de monomères éthyléniques à cycle lactame, par exemple de vinylactames avec les composés suivants en tant qu'agents de transfert de chaîne :
- diphényldithiocarb amate de diéthylmalonate (DPCM) : 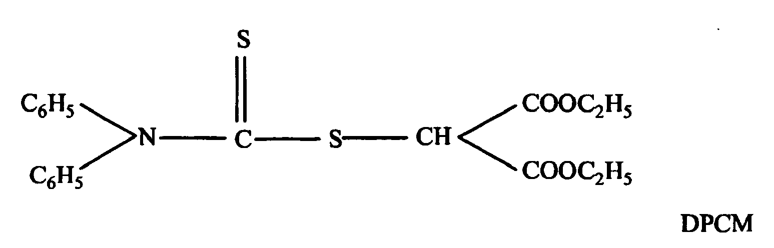
- diphényl dithiocarbamate de fluoroacétate d'éthyle (DPFEM) 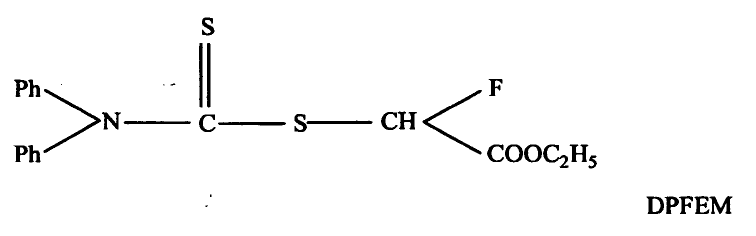
- xanthate : C₂H₅ O C(S)S C H (CH₃) COOCH₃

### Matières premières

- Le monomère éthylénique à cycle lactame tel que la N-vinylpyrrolidone (VP) ou la N-vinylcaprolactame (VCap) est acquis auprès de la Société ALDRICH® et est distillé sous vide avant utilisation.
- Les autres monomères, tel que acrylate de tertiobutyle, méthacrylate de méthyle, sont acquis auprès de la Société ALDRICH® et sont séchés sur hydrure de calcium et distillés sous vide.
- L'AIBN est acquis auprès de la Société ALDRICH® et est recristallisé dans le méthanol.
- La diphénylamine, le CS₂, et le bromodiéthylmalonate sont acquis auprès de la Société ALDRICH® et sont utilisés tels qu'ils sont reçus.
- Les solvants tels que le dioxane sont séchés par distillation sur CaH₂ avant utilisation.

### 2. 1. Synthèse de l'agent de transfert de chaîne.

### 2. 1. 1. : Synthèse du diphényldithiocarbamate de diéthyl malonate (DPCM)

Le diphényldithiocarbamate diéthylmalonate (DPCM) est synthétisé à partir de la diphénylamine et du malonate de bromodiéthyle de la manière suivante :

A une solution de 0,625 g de NaH (purifié par lavage avec de l'hexane sec) dans 5 ml de THF (sec), on ajoute à 0°C 1,69 g de diphénylamine (10 mmol) dans 10 ml de DMSO et 5 ml de THF. Le mélange réactionnel est agité pendant 1,5 heures pour donner une solution verte limpide. A cette solution, on ajoute 1,2 éq de CS₂ (1,42 ml, 1,2 mmol), et on agite pendant 30 minutes à 0°C pour obtenir une solution jaune orangée du sel de sodium du diphényldithiocarbamate.

On ajoute du malonate de bromodiéthyle (10 mmol) à la solution ci-dessus à -20°C et on amène lentement la température du mélange réactionnel jusqu'à la température ambiante. Après agitation pendant 2 heures à température ambiante, le mélange réactionnel est traité avec de l'eau et extrait avec de l'éther. La couche éthérée est séchée sur du MgSO₄ et concentrée.

Rendement : 51 %.

La pureté du produit a été vérifiée par RMN.

### 2. 1. 2. Synthèse du diphényl dithiocarbamate d'acétate de fluoroéthyle (DPFEA)

On place de l'hydrure de sodium (7 mmol/0,168 g, 1,3 eq dans 5 ml THF) dans un flacon séché à la flamme et on l'agite à 0°C. A ce mélange, on ajoute goutte à goutte de la diphénylamine (5,4 mmoles, 1 eq) dans 5 ml de THF, et 10 ml de DMSO et on agite pendant une heure. On ajoute du disulfure de carbone (2,3 eq) à la solution de 0°C et on l'agite pendant encore une demi-heure. On abaisse la température de la solution jusqu'à 18°C et on ajoute l'équivalent d'acétate de fluoroéthyle. Après addition, on élève lentement la température du mélange réactionnel jusqu'à la température ambiante et on l'agite pendant une demi-heure à température ambiante. Le produit obtenu est hydrolysé en ajoutant de l'eau et la couche organique est extraite avec de l'éther. L'extrait éthérée est concentré pour donner des cristaux jaunes de (DPFEA) et la pureté du produit est vérifiée par analyse RMN.

### 2. 2. Polymérisation

### 2. 2. 1.

Le mode opératoire général pour la polymérisation est le suivant que l'agent de transfert de chaîne soit le diphényl dithiocarbamate de diéthylmalonate ou bien le dithiocarbamate d'acétate de fluoréthyle.

Dans ce qui suit, la vinylpyrrolidone est mentionnée, mais le procédé de préparation peut être généralisé à tout monomère éthylénique à cycle lactame.

### Première étape

### Préparation du précurseur (ou macroamorceur ): copoly(vinylpyrrolidone / monomère minoritaire)

La polymérisation de la vinylpyrrolidone (VP) et d'un monomère minoritaire (par exemple l'acrylate de tertiobutyle) (séquence A) utilisant le diphényl dithiocarbamate de malonate de diéthyle en tant que réactif RAFT est réalisée en mettant en oeuvre l'AIBN en tant qu'amorceur.

Dans une expérience typique, la VP, le monomère minoritaire de la séquence A (à savoir l'acrylate de tertiobutyle), le diphényl dithiocarbamate de malonate de diéthyle (rapport agent de transfert de chaîne/monomères = 1/100), l'AIBN (10 % de l'agent de transfert de chaîne) et du dioxane (rapport de 1/1 en volume par rapport au monomère) sont placés dans un tube Schlenk. Le mélange réactionnel est dégazé par trois cycles de congélation-mise sous vide-décongélation (« freeze-pump-thaw cycle »), puis fermé hermétiquement sous vide et chauffé dans un bain à température constante à 80°C.

Le macroamorceur « actif » que l'on peut représenter par « X' -copoly(vinylpyrrolidone / monomère minoritaire)-X » avec X = (phényl)₂NC(S)S-, et X' = -CH(COOC₂H₅)₂, obtenu est purifié par précipitations répétées dans l'éther diéthylique et est séché sous vide.

### Deuxième étape

### Polymérisation de la seconde séquence à l'extrémité du macroamorceur « actif » « X'-copoly(vinylpyrrolidone / monomère minoritaire)-X »

La polymérisation du second monmère formant la séquence B (qui est par exemple le méthacrylate de méthyle) a lieu en présence du macroamorceur «X' - copoly(vinylpyrrolidone / monomère minoritaire)-X » ci-dessus (séquence A), dans 1,5 ml de dioxane et en présence de AIBN (0,1 mol % par rapport au total du monomère et du macroamorceur).

Le mélange réactionnel est soumis à trois cycles de congélation - mise sous vide-décongélation et chauffé dans un bain d'huile sous agitation à 80°C pendant 20 heures.

Une fois la réaction terminée, le mélange réactionnel est dissous dans le dichlorométhane (quantité juste nécessaire à la dissolution) et il est précipité dans l'éther.

La solution éthérée trouble est filtrée et concentrée par évaporation instantanée « flash » et est ajoutée à du pentane ; le précipité obtenu est séché sous vide à environ 70°C pendant 8 heures.

### 2. 2. 2.

En variante, la polymérisation peut être réalisée de la manière suivante : on effectue d'abord la synthèse d'un macroamorceur poly(méthacrylate de méthyle) (séquence B) par exemple, puis on effectue la polymérisation de la séquence (A) constituée de vinylpyrrolidone et du monomère minoritaire tel que l'acrylate de tertiobutyle à l'extrémité de ce macroamorceur. On suit le même mode opératoire que dans le paragraphe 2. 2. 1.

### 2. 2. 3. Polymérisation en utilisant un xanthate comme agent de transfert de chaîne : synthèse sans étape de purification intermédiaire (« one pot synthesis », en anglais)

De la N-vinylpyrrolidone (4 mol, 3,74 ; 10⁻² moles) (le mode opératoire peut s'appliquer aussi à un autre monomère éthylénique à cycle lactame), le monomère minoritaire : l'acrylate de tertiobutyle, l'agent de transfert de chaîne (xanthate ci-dessus, 0,0839 g, rapport agent de transfert de chaîne/monomère=environ 1/100 en moles) et du dioxane (4 ml) sont placés dans un ballon préséché, et de l'AIBN (0,0061 g, 10 % en moles de l'agent de transfert de chaîne) y est ajouté sous azote.

Le mélange réactionnel est soumis à trois cycles de congélation-mise sous vide-décongélation et est chauffé dans un bain d'huile thermostaté à 80°C sous agitation. La réaction est arrêtée après 14 heures par refroidissement dans l'azote liquide.

Le second monomère (formant la séquence B), par exemple le méthacrylate de méthyle (3 ml), et du dioxane (3 ml) sont ajoutés à ce mélange réactionnel à température ambiante sous azote. Le mélange réactionnel est de nouveau soumis à trois cycles de congélation-mise sous vide-décongélation et est chauffé à 80°C pendant encore 24 heures.

Une fois la réaction terminée, le mélange réactionnel est dissous dans le dichlorométhane (quantité juste nécessaire à la dissolution) et il est précipité dans l'éther. La solution éthérée trouble est filtrée et concentrée par évaporation instantanée (« flash ») et elle est ajoutée à du pentane, le précipité obtenu est séché sous vide à environ 70°C pendant 8 heures.

### Caractérisations

- La conversion est mesurée par pesée du polymère.
- La masse du macroamorceur à base de vinyllactame, par exemple le copoly(vinylpyrrolidone / acrylate de tertiobutyle), est déterminée par chromatographie en phase gazeuse GPC (appareil Varian 9012®) avec des colonnes gels G4000 G3000 G2500 de TSK®, équipées d'un détecteur infrarouge VARIAN® RI 4. L'éluant est un mélange 80/20 eau méthanol contenant 0,1 M de nitrate de sodium. Le débit de l'éluant est de 0,5 ml/min. La calibration est effectuée par des étalons poly(oxyde d'éthylène).
   La mesure de masse n'est donc qu'une mesure comparative et les valeurs analogues ne représentent pas les masses moléculaires réelles. Cependant, les résultats peuvent être utilisés pour noter la tendance dans l'évolution des masses moléculaires et également pour déterminer la dispersité en masse des chaînes.
- La masse du macroamorceur PMMA est déterminée par GPC (Varian® 9012) avec des colonnes gels G4000 G3000 G2500 de TSK®, équipées d'un détecteur infrarouge Varian® RI 4. L'éluant est le THF. L'étalon est le polystyrène.
- La masse globale du copolymère est déterminée par chromatographie GPC en phase solvant (Varian® 9012) avec des colonnes gels G4000 G3000 G2500 de TSK®, équipées d'un détecteur infrarouge Varian® RI 4. L'éluant est le THF. L'étalon est le polystyrène.
- La proportion des différentes séquences est déterminée par RMN¹H (Bruker®, 200 MHz) en faisant le rapport des surfaces des pics correspondants aux monomères des différentes séquences (à savoir par exemple d'une part MMA et d'autre part VP).

Après avoir décrit ci-dessus le mode opératoire général pour la synthèse des copolymères selon l'invention, on donne ci-après des exemples concernant la préparation d'un copolymère spécifique conforme à l'invention (exemple 1) ainsi que deux exemples comparatifs.

### Exemple comparatif 1

Dans cet exemple, on prépare un copolymère (non-conforme à l'invention) comprenant une séquence polyvinylpyrrolidone et une séquence poly(méthacrylate de méthyle) : (PVP) 90 %-b-(PMMA) 10 % selon le procédé 1 (« ATRP » inverse) décrit ci-dessus.

### Première étape

### Synthèse du macroamorceur polyvinylpyrrolidone-Br : PVP-Br

Du CuBr₂ (0,0314 g, 1,4 x 10⁻⁴ moles) et du Me₆TREN (0,0970 g, 4,2 x 10⁻⁴ moles) sont placés dans un ballon de 50 mL et on ajoute 5 mL de dioxane.

On ajoute ensuite de la N-vinylpyrrolidone (NVP) (5,2 g, 0,0468 moles) suivie par de l'AIBN (azobisisobutyronitrile) (0,0154 g, 9,36 x 10⁻⁵ moles).

Le rapport dioxane/monomère est de 1/1 en volume.

Les proportions AIBN/CuBr₂/CuO/Me₆TREN sont : 1/1,5/0,15/3.

La réaction est réalisée à une température de 80°C pendant 3 heures 45. La conversion atteint 97 %.

### Deuxième étape

### Synthèse du copolymère : Polymérisation du méthacrylate de méthyle (MMA) à l'extrémité du macroamorceur polyvinylpyrrolidone (PVP-Br)

On ajoute le macroamorceur de PVP(PVP-Br) dans du CuBr (rapport PVP-Br/CuBr 1 :1,5) et du Me₆TREN (CuBr/Me₆TREN 1:3) dans le dioxane sous atmosphère d'azote.

Macroamorceur polyvinylpyrrolidone PVP-Br : 0,041 mM : 0,92 g.

Méthacrylate de méthyle (MMA) : 4,09 mM : 0,4 g.

Solvant : Dioxane : 1,5 ml.

La réaction est réalisée à une température de 100°C pendant 14 heures.

La conversion est de 51 %.

Les caractéristiques du macroamorceur et du copolymère final sont résumées dans le tableau suivant :

| Copolymère | VP/AIBN | Mn (macroamorceur PVP) (GPC) | % PVP/poids total RMN | % PMMA/poids total RMN | Mn PMMA déduite par RMN |
|---|---|---|---|---|---|
| (PVP) 90 %--b-- (PMMA) 10 % | 1/0,35 | 24 400 | 87,5 % | 12,5 % | 9600 |

| | | | | | |
|---|---|---|---|---|---|
| Vinylpyrrolidone = VP Azobisisobutyronitrile = AIBN Mₙ = masse moléculaire moyenne en nombre | | | | | |

### Exemple comparatif 2

Dans cet exemple, on prépare un copolymère (non-conforme à l'invention) comprenant une séquence polyvinylpyrrolidone (PVP) et une séquence polymère Polystyrène (PS) : (PVP) 80%-b-(PS) 20%.

Le polymère recherché est préparé par un procédé analogue à celui décrit dans le paragraphe 2.2.2 ci-dessus, les monomères étant adaptés pour obtenir le polymère voulu.

### Première étape

### Synthèse du macroamorceur polystyrène

Agent de transfert de chaîne : DPCM

Rapport Monomère/Agent de transfert de chaîne : 200 (en moles)

Solvant : Dioxane

Température de la réaction : 100°C

### Deuxième étape

### Polymérisation de la seconde séquence à l'extrémité du macroamorceur polystyrène

Le mélange réactionnel comprend 0,5 g du macroamorceur polystyrène préparé ci-dessus et 0,25 g de vinylpyrrolidone dans 1,5 ml de dioxane et la réaction est réalisée en présence d'AIBN (0,1 mol% par rapport au styrène).

La conversion est de 50%

Les caractéristiques du copolymère final sont résumées dans le tableau suivant :

| Mn Polystyrène (NMR) | % PVP/Poids total RMN | % PS/moles total RMN | % PS/poids total RMN | Mn de PVP déduit par (RMN) | Mn Mw Mp (GPC) |
|---|---|---|---|---|---|
| 4150 | 79 | 29 | 21 | 15330 | Mw 40500 Mn 22700 Mp 41300 Ip 1,8 |

| | | | | | |
|---|---|---|---|---|---|
| Mₙ = masse moléculaire moyenne en nombre | | | | | |

### Exemple 1

Dans cet exemple, on prépare un copolymère (conforme à l'invention) comprenant une séquence A (80 % en poids) constituée par un copolymère à base de 70 % de vinylpyrrolidone (PVP) et 30 % d'acrylate de t-butyle, et une séquence B (20 % en poids) constituée par du poly(méthacrylate de méthyle).

Le polymère recherché est préparé par un procédé analogue à celui décrit dans le paragraphe 2.2.3. ci-dessus. Le mélange de monomères étant adapté pour obtenir le polymère voulu.

On place de la N-vinylpyrrolidone (VP) et de l'acrylate de tertiobutyle (3,74 x 10⁻² moles au total), l'agent de transfert de chaîne (xanthate décrit plus haut, 0,0839 g, rapport agent de transfert de chaîne/monomères : environ 1/100) et du dioxane (4 ml) dans un flacon séché au préalable ; et de l'AIBN (0,0061 g, 10 % en moles par rapport à l'agent de transfert de chaîne) est ajouté dans le flacon sous azote.

Ce mélange réactionnel est soumis à trois cycles congélation-mise sous vide-décongélation et il est chauffé dans un bain d'huile thermostaté à 80°C sous agitation.

La réaction est arrêtée après 14 heures par refroidissement dans l'azote liquide.

Le monomère de la seconde séquence (méthacrylate de méthyle) (3 ml) et du dioxane (3 ml) sont ajoutés à ce mélange réactionnel à température ambiante sous azote. Le mélange réactionnel est de nouveau soumis à trois cycles congélation-mise sous vide-décongélation et est chauffé à 80°C pendant encore 24 heures.

Une fois la réaction terminée, le mélange réactionnel est dissous dans du dichlorométhane (quantité juste nécessaire pour la dissolution) et il est précipité dans l'éther

La solution éthérée trouble est filtrée et concentrée par évaporation instantanée (« flash ») et elle est ajoutée à du pentane ; le précipité obtenu est séché sous vide à environ 70°C pendant huit heures, et il est caractérisé par GPC et RMN.

Les caractéristiques des réactifs et du procédé sont résumées ci-après.

Agent de transfert de chaîne : xanthate C₂H₅OC (S) SCH (CH₃)COOCH₃.

Rapport molaire : xanthate/monomères : 1/100.

Rapport AIBN/agent de transfert de chaîne xanthate : 10 % en moles.

Rapport monomères/AIBN : 0,1.

Température de la réaction : 80°C.

Solvant : dioxane.

Vinylpyrrolidone (VP) : 0,027 moles, soit 3 g.

Acrylate de tertiobutyle (AtBu) : 0,0098 moles, soit 1,254 g

Les caractéristiques du copolymère sont résumées dans le tableau suivant :

| Copolymère | VP/AtBu en poids | Mn [poly(VP co AtBu)] mesurée par GPC | Conversion du MMA | % PNMA/poids total | Mn PMMA déduit par RMN | % [poly (VP co AtBu)]/poids total |
|---|---|---|---|---|---|---|
| Poly(VP : 70 % : acrylate de t-butyle : 30 %) 80 %-b-(polyméthacrylate de méthyle) 20 % | 70/30 | 6 500 | 90 % | 25 | 2 160 | 75 |

| | | | | | | |
|---|---|---|---|---|---|---|
| Mn : masse moléculaire moyenne en nombre. | | | | | | |

### Exemple 2

Dans cet exemple, on réalise une mesure du collant des copolymères préparés dans les exemples comparatifs 1 et 2 et dans l'exemple 1.

Un film de polymère est réalisé à partir d'une solution dans l'éthanol, sur un support de verre. Le polymère est dissous à 10% dans le solvant.

Le film est séché dans une enceinte à humidité contrôlée.

Le collant est évalué par contact avec le doigt sur une échelle de 0 à 10, à savoir : 0 : pas de collant, 5 : collant moyen, 10 : collant élevé.

### Exemple 1 :

valeur de 2 à HR ambiante,
valeur de 4 à HR : 100

### Exemple comparatif 1 :

valeur de 5 à HR ambiante
valeur de 10 à HR : 100

### Exemple comparatif 2 :

valeur de 4 à HR ambiante
valeur de 8 à HR : 100

Le polymère de l'exemple 1 conforme à l'invention présente un collant inférieur aux polymères des exemples comparatifs non-conformes à l'invention aussi bien dans des conditions d'humidité ambiante que dans des conditions d'humidité élevée (HR : 100).

### Exemple 3

Dans cet exemple, on formule un spray avec le polymère conforme à l'invention de l'exemple 1.

Le polymère de l'exemple 1 est dissous dans l'éthanol à 6%.

Le polymère est appliqué en spray sur la chevelure.

La coiffure ne présente pas de collant, même à taux élevé d'humidité (HR > 50%) .

## Revendications

1. Copolymère éthylénique, séquencé, linéaire comprenant :
- au moins une séquence A susceptible d'être préparée à partir de monomères, constitués d'un monomère éthylénique à cycle lactame répondant à la formule (I) suivante : 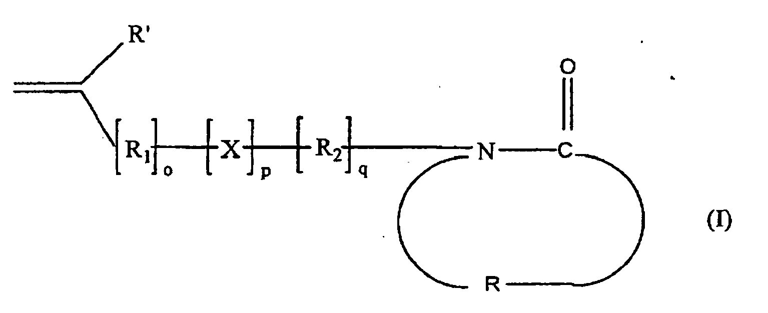 dans laquelle :
• R représente un groupe - (CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
• R' représente H ou un groupe méthyle ;
• R₁ et R₂, qui peuvent être identiques ou différents représentent un groupe alkylène ou aralkylène linéaire, ramifié ou cyclique de 1 à 22 C, dans lequel un ou plusieurs des atomes de carbone sont éventuellement remplacés par un atome d'oxygène ou d'azote ;
• X est choisi parmi -OCO-, -NHCO-, -COO-, -O- ;
• O, p, q, représentent chacun indépendamment les uns des autres 0 ou 1 ;
et d'au moins un monomère non hydrophile , à savoir un monomère dont l'homopolymère préparé à partir de ce monomère n'est pas hydrosoluble on hydrodispersible; le pourcentage de monomère éthylénique à cycle lactame de formule (I) dans la séquence A étant de 60 à 80% en poids ;
- et au moins une séquence B susceptible d'être préparée à partir de monomères ne comportant pas de monomère éthylénique à cycle lactame de formule (I) ou comportant une proportion minoritaire de celui-ci.

2. Copolymère selon la revendication 1, dans lequel dans la formule (I) , o est 0, p est 1, q est 1, R₂ représente -CH₂CH₂-, X représente COO ou CONH, et R est (CH₂)₃ ou (CH₂)₅ ou (CH₂CH₂NH) .

3. Copolymère selon la revendication 1, dans lequel le monomère éthylénique à cycle lactame est un vinyllactame qui répond à la formule (II) suivante : 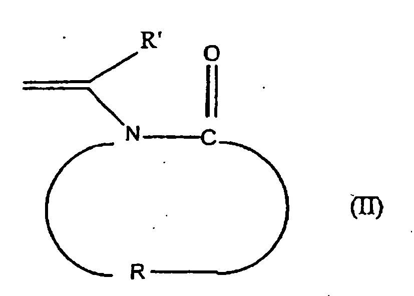 dans laquelle R, R' ont la signification déjà donnée dans la revendication 1.

4. Copolymère selon l'une quelconque des revendications 1 à 3, dans lequel dans les formules (I) et (II), R représente -(CH₂)ₙ-, où n est un nombre enter de 3 à 5, ou bien R représente -CH₂-CH₂-NH-.

5. Copolymère selon l'une quelconque des revendications précédentes, qui est un copolymère filmogène.

6. Copolymère selon l'une quelconque des revendications précédentes, qui a une masse moléculaire moyenne en nombre de 4 000 à 1 000 000, de préférence de 4 000 à 800 000, de préférence encore de 4 000 à 500 000.

7. Copolymère selon l'une quelconque des revendications précédentes, dans lequel la séquence A, représente de 1 à 99 % en poids du copolymère, de préférence de 10 à 95 %, de préférence encore de 20 à 90 %, en poids du poids total du copolymère.

8. Copolymère selon l'une quelconque des revendication précédentes, dans lequel la séquence B représente de 1 à 99 % en poids du copolymère, de préférence de 5 à 90 % en poids, de préférence encore de 10 à 80% en poids du poids total du copolymère.

9. Copolymère selon l'une quelconque des revendications précédentes, dans lequel la masse moléculaire de chaque séquence A ou B est comprise entre 2000 et 1 000.000, de préférence comprise entre 2000 et 800 000, de préférence encore comprise entre 2000 et 500 000.

10. Copolymère selon l'une quelconque des revendications précédentes, dans lequel lesdits monomères à partir desquels est préparée ladite séquence A comprennent une proportion de monomère éthylénique à cycle lactame, par exemple de vinyllactame, de formule (I) ou (II), de 65 à 75% en poids, par exemple de 70% en poids.

11. Copolymère selon l'une quelconque des revendications précédentes, dans lequel ladite séquence A a une température de transition vitreuse globale de la séquence comprise entre 0 et 250°C, de préférence entre 0 et 220°C, et de préférence encore entre 5 et 200°C.

12. Copolymère selon la revendication 1 dans lequel ledit N-vinyllactame de formule (I) est le pyrrilidino-éthylacrylate ou le pyrrilidino-éthylméthacrylate.

13. Copolymère selon l'une quelconque des revendications 3 à 12, dans lequel ledit N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

14. Copolymère selon l'une quelconque des revendications précédentes, dans lequel la séquence A est un copolymère statistique, alterné où à gradient.

15. Copolymère selon l'une quelconque des revendications précédentes, dans lequel lesdits monomères non hydrophiles de la séquence A sont choisis parmi les monomères suivants :
- Les hydrocarbures éthyléniques de 2 à 10 C, tels que l'éthylène, l'isoprène, ou le butadiène ;
- Les acrylates de formule CH₂ = CHCOOR₃ ;
- Les méthacrylates de formule : 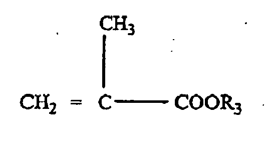 dans lesquelles R₃ représente :
• un groupe alkyle linéaire, ou ramifié, de 1 à 18 atomes de carbone, dans lequel se trauve(nt) éventuellement intercalé(s) un ou plusieurs hétéroatomes choisis parmi 0, N, S et P,
ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle,
• un groupe cycloalkyle en C₃ à C₁₂, tel que le groupe isobornyle,
• un groupe aryle en C₃ à C₂₀ tel que le groupe phényle,
• un groupe aralkyle en C₄ à C₃₀ (groupe alkyle en C₁ à C₈) tel que 2- phényl-éthyle ou benzyle,
• un groupe hétérocyclique de 4 à 12 chaînons contenant un ou plusieurs hétéroatomes choisis parme O, N, et S, le cycle étant aromatique ou non,
• un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé (s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆, ou un groupe phényle,
• des exemples de groupes R₃ sont les groupes méthyle, éthyle, propyle, isobutyle, n-butyle, tertiobutyle, héxyle, éthylhéxyle, octyle, lauryle, isooctyle, isodécyle, dodécyle, cyclohexyle, t-butylcyclohexyle, t-butylbenzyle, isobornyle, phényle, furfurylméthyle, tétrahydrofurfurylmethyle, éthyl-2-perfluorohexyle,
• un autre exemple de groupe R₃ pour les acrylates sont les groupes R₃ = - (OC₂H₄)ₘ-OR'', avec m = 5 à 150 et , R" = H ou alkyle de C₆ à C₃₀, par exemple -POE- lauryle ;
- Les (méth)acrylamides de formule : 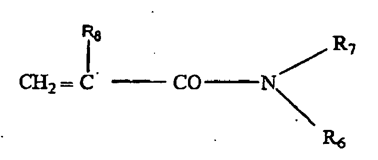 où
R₈ désigne H ou méthyle,
et R₇ et R₆ identiques ou différents représentent chacun un atome d'hydrogène ou un groupe alkyle de 1 à 18 atomes de carbone linéaire ou ramifié, dans lequel se trouve(nt), éventuellement intercalé(s) un ou plusieurs hétéroatomes choisis parmi 0, N, S et P, ledit groupe allyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents, représentent un groupe alkyle en C₁ à C₆, ou un groupe phényle,
des exemples de ces groupes sont les groupes méthyle, éthyle, n-butyle, t-butyle, isopropyle, isohexyle, isooctyle, isononyle,
• un groupe cycloalkyle en C₃ à C₁₂, tel que le groupe isobornyle,
• un groupe aryle en C₃ à C₂₀ tel que phényle,
• un groupe aralkyle en C₄ à C₃₀ (groupe alkyle en C₁ à C₈) tel que 2- phényl éthyle ou benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle dé 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅), où R₄ et R₅ identiques ou différents représentent un groupe alkyle en C₁ à C₆, ou un groupe phényle,
des exemples de monomères (méth)acrylamide sont le N-éthyl(méth)acrylamide, le N-butylacrylamide, le N-t-butylacrylamide, le N-isopropylacrylamide, le N,N-diméthyl(méth)acrylamide, le N-octylacrylamide, le N-dodécylacrylamide, et l'undécylacrylamide ;
- Les composés allyliques de formule : CH₂ = CH-CH₂-R₉ ou CH₂ = C(CH₃) -CH₂-R₉ ;
- Les composés vinyliques de formule : CH₂ = CH-R₉,
où R₉ est un groupe :
• OR₁₀, où R₁₀ représente un groupe phényle ou un groupe alkyle en C₁ à C₁₂ (le monomère est un éther de vinyle ou d'allyle),
• OCOR₁₁, où R₁₁ représente :
• un groupe alkyle de 2 à 12 C linéaire ou ramifié (le monomère est un ester de vinyle ou d'allyle) ;
• un groupe cycloalkyle en C₃ à C₁₂ tel que isobornyle, cyclohexyle,
• un groupe aryle en C₃ à C₂₀ tel que phényle,
• un groupe aralkyle en C₄ à C₃₀ (groupe alkyle en C₁ à C₈) tel que 2-phényléthyle, benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅) où R₄ et R₅ identiques ou différents représentent un groupe alkyle en C₁ à C₆, ou un groupe phényle,
des exemples de monomères vinyliques sont le vinylcyclohexane, et le styrène,
des exemples d'esters de vinyle sont : l'acétate de vinyle le propionate de vinyle, le butyrate de vinyle, l'éthylhexanoate de vinyle, le néononanoate de vinyle, et le néododécanoate de vinyle, parmi les éthers de vinyle on trouve par exemple le vinyl méthyl ether, le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

16. Copolymère selon l'une quelconque des revendications précédentes, dans lequel le ou les monomères, autres que le monomère éthylénique à cycle lactame, à partir duquel ou desquels est susceptible d'être préparée la séquence A, sont, de préférence, choisis parmi les monomères dont la température de transition vitreuse Tg de l'homopolymère correspondant est basse, de préférence encore inférieure ou égale à 50°C, mieux inférieure ou égale à 20°C, encore mieux inférieure ou égale à 0°C.

17. Copolymère selon la revendication 16, dans lequel le ou les monomères, autres que le monomère éthylénique à cycle lactame, ont, en outre, une Tg de l'homopolymère correspondant supérieure ou égale à -150°C.

18. Copolymère selon la revendication 15, dans lequel le ou les monomère (s), autres que le monomère éthylénique à cycle lactame, à partir duquel ou desquels la séquence A est susceptible d'être préparée est (sont) choisi(s) parmi les monomères suivantes dont la température de transition vitreuse Tg de l'homopolymère correspondant est inférieure ou égale à 50°C, tels que : l'acrylate de méthyle (Tg=10°C), l'acrylate d'éthyle (Tg=-24°C), l'acrylate de n-butyle (Tg=-54°C), l'acrylate de t-butyle (Tg=43°C) l'acrylate d'éthylhexyle (Tg=-50°C), l'acrylate d'isobutyle (-24°C), le méthacrylate de butyle (Tg=20°C), le méthacrylate de n-hexyle, le méthacrylate de POE (n=8 à 10) (Tg=-55°C), l'acétate de vinyle (Tg=23°C) ; et d'autres monomères, tels que le méthacrylate de méthyle (Tg=105°C), le méthacrylate d'éthyle, le méthacrylate d'isobutyle, l'acrylate de furfuryle, l'acrylate d'isobornyle, l'acrylate de tertiobutylcyclohexyle, le styrène, et le vinylcyclohexane.

19. Copolymère selon l'une quelconque des revendications précédentes, dans lequel la ou les séquences B autres que la séquence A est(sont) susceptible(s) d'être préparée(s) à partir du ou de plusieurs monomères éthyléniques choisis parmi : les monomères allyliques, les acrylates, les méthacrylates, les acrylamides, les méthacrylamides, les monomères vinyliques, et leurs mélanges, et éventuellement des monomères éthyléniques à cycle lactame, par exemple des monomères de vinyllactame, de formule (I) ou (II), la proportion en poids desdits monomères (I) ou (II) étant inférieure à 50 % en poids, de préférence inférieure ou égale à 45 % en poids, de préférence encore, inférieure ou égale à 40 % en poids, et mieux inférieure ou égale à 30% en poids.

20. Copolymère selon l'une quelconque des revendications précédentes, dans lequel le ou lesdits monomères, à partir duquel ou desquels est susceptible d'être préparée la séquence B, est(sont) choisi(s) parmi les monomères non hydrophiles et les monomères hydrophiles.

21. Copolymère selon l'une quelconque des revendications précédentes, dans lequel la séquence B a une température de transition vitreuse inférieure ou égale à 50°C, de préférence inférieure ou égale à 20°C, de préférence encore inférieure ou égale à 0°C.

22. Copolymère selon l'une quelconque des revendications précédentes, dans lequel le ou les monomère(s) à partir duquel ou desquels est(sont) susceptible(s) d'être préparée la(les) séquence(s) B sont choisis parmi les monomères dont la température de transition vitreuse Tg de l'homopolymère correspondant est basse, de préférence encore inférieure ou égale à 50°C, mieux inférieure ou égale à 20°C, et encore mieux inférieure ou égale à 0°C.

23. Copolymère selon la revendication 22, dans lequel le ou lesdits monomère(s) ont, en outre, une Tg de l'homopolymère correspondant supérieure à -150°C.

24. Copolymère selon la revendication 20, dans lequel les monomères hydrophiles sont choisis parmi les monomères ioniques tels que les monomères cationiques, les monomères anioniques et les bétaïnes ; les monomères non ioniques ; et les monomères pouvant être rendus hydrophiles par suite d'une hydrolyse.

25. Copolymère selon la revendication 20, dans lequel les monomères non hydrophiles sont choisis parmi les monomères tels que définis dans les revendications 15 à 18.

26. Copolymère selon la revendication 24, dans lequel les monomères cationiques sont choisis parmi la 2-vinylpyridine ; la 4-vinylpyridine, le (méth)acrylate de diméthylaminoéthyle ; le (méth)acrylate de diéthylaminoéthyle ; le diméthylaminopropyl(méth)acrylamide ; et les formes salifiées ou quaternisées de ceux-ci, qu'il s'agisse de sels d'acides minéraux, tels que l'acide sulfurique ou l'acide chlorhydrique, ou de sels d'acides organiques.

27. Copolymère selon la revendication 24, dans lequel les monomères anioniques sont choisis parmi l'acide acrylique, l'acide méthacrylique, l'avide crotonique, l'anhydride maléique, l'acide itaconique, l'acide fumarique, l'acide maléique ; l'acide styrénesulfonique, l'acide 2-acrylamido-2-méthylpropanésulfonique, l'acide vinylsulfonique, l'acide vinylbenzoïque, le méthacrylate de sulfopropyle, l'acide vinylphosphonique, et les sels de ceux-ci.

28. Copolymère selon la revendication 24, dans lequel les monomères ioniques de type bétaïne sont choisis parmi :
- les carboxybétaïnes ou sulfobétaïnes éthyléniques obtenues, par exemple par quaternisation de monomères à insaturation éthylénique comportant une fonction amine par des sels d'acide carboxylique à halogène mobile, par exemple, le chloroacétate de sodium, ou par des sulfones cycliques, par exemple la propanesulfone.

29. Copolymère selon la revendication 24, dans lequel les monomères non-ioniques sont choisis parmi :
- les (méth)acrylates ou (métha)crylamides d'hydroxyalkyle dont le groupe alkyle a de 2 à 4 atomes de C, en particulier le (méth)acrylate d'hydroxyéthyle ou d'hydroxypropyle ;
- les (méth)acrylates ou (métha)crylamides d' alcoxy (C₁₋₄) alkyle (C₁₋₄) tel que méthoxyéthyle, éthoxyéthyle et méthoxypropyle,
- les (méth)acrylates ou (métha)crylamides à groupe -(OC₂H₄)ₘ-OR''', avec m = 5 à 150 et, R''' = H ou alkyle de C₁ à C₄, par exemple -POE- méthoxy ;-POE-OH ;
- les vinyllactames,
- et les (méth)acrylates de polysaccharide comme l'acrylate de saccharose.

30. Copolymère selon l'une quelconque des revendications précédentes, choisi parmi :
- les copolymères biséquencés (AB) ;
- les copolymères triséquencés (ABA, BAB, ABC, ACB), avec C différent de A ou B,
- les copolymères multiséquencés ayant plus de trois séquences : (AB)ₙ, (ABA)ₙ, (BAB)ₙ, (ABC)ₙ, (ACB)ₙ, avec C différent de A ou B ou les copolymères multiséquencés ayant plus de trois séquences différentes, de type ABCD.

31. Composition cosmétique ou pharmaceutique comprenant le copolymère selon l'une quelconque des revendications 1 à 30.

32. Composition selon la revendication 31, contenant de 0,1 à 60 % en poids, de préférence de 0, 5 % à 50 % en poids, et de préférence encore de 1 à 40 % en poids du copolymère.

33. Composition selon l'une quelconque des revendications 31 et 32, comprenant outre ledit copolymère un milieu physiologiquement acceptable, dans lequel le copolymère se trouve sous forme dissoute ou dispersée.

34. Composition selon l'une quelconque des revendications 31 à 33, dans laquelle le milieu physiologiquement acceptable comprend un ou plusieurs solvants appropriés formant une phase hydrophile choisis parmi l'eau et les mélanges d'eau et de solvant(s) organique(s) hydrophile(s), tels que les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols.

35. Composition selon la revendication 34, dans laquelle la phase hydrophile contient, en outre, des éthers en C₂.

36. Composition selon l'une quelconque des revendications 31 à 35, dans laquelle ledit milieu physiologiquement acceptable comprend, en outre, une phase grasse notamment constituée de corps gras liquides à température ambiante, et/ou de corps gras solides à température ambiante, d'origine animale, végétale, minérale ou synthétique.

37. Composition selon l'une quelconque des revendications 31 à 36, dans laquelle le milieu physiologiquement acceptable comprend, en outre, un ou plusieurs solvants organiques cosmétiquement et/ou pharmaceutiquement acceptables.

38. Composition selon l'une quelconque des revendications 31 à 37, dans laquelle ledit milieu physiologiquement acceptable comprend, en outre, un ou plusieurs agents auxiliaires de filmification choisis parmi les agents plastifiants et les agents de coalescence.

39. Composition selon l'une quelconque des revendications 31 à 38, dans laquelle le milieu physiologiquement acceptable comprenant, en outre, une ou des matières colorantes choisies parmi les colorants hydrosolubles, et les matières colorantes pulvérulentes, comme les pigments, les nacres et les paillettes.

40. Composition selon l'une quelconque des revendications 31 à 39, dans laquelle le milieu physiologiquement acceptable comprend, en outre, une ou plusieurs charges.

41. Composition selon l'une quelconque des revendications 31 à 40, dans laquelle le milieu physiologiquement acceptable comprend, en outre, un ou plusieurs ingrédient(s) couramment utilisé(s) en cosmétique et/ou en pharmacie, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les polymères hydrosolubles, liposolubles ou en dispersion dans l'eau ou dans une phase aqueuse, fibrogènes ou non, ou lieurs mélanges.

42. Composition selon lune quelconque des revendications 31 à 41, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre.

43. Composition selon l'une quelconque des revendications 31 à 42, **caractérisée par le fait qu'**il s'agit d'un produit capillaire, tel qu'une laque ou un shampooing.

44. composition selon l'une quelconque des revendications 31 à 42, **caractérisée par le fait qu'**il s'agit d'une composition de maquillage, telle qu'un vernis à ongles.

45. Procédé cosmétique de maquillage ou de soin des matières kératiniques comprenant l'application sur les matières kératiniques d'une composition selon l'une des revendications 31 à 44.

46. Utilisation du copolymère selon l'une quelconque des revendications 1 à 30, pour améliorer le pouvoir coiffant et/ou la tenue, sans collant notamment au toucher, d'une laque pour cheveux.

47. Utilisation du copolymère selon l'une quelconque des revendications 1 à 30, pour augmenter l'adhérence et la résistance à l'usure, sans collant notamment au toucher d'un vernis à ongles.

48. Utilisation du copolymère selon l'une quelconque des revendications 1 à 30, pour améliorer la tenue d'une composition de maquillage.

49. Utilisation du copolymère selon l'une quelconque des revendications 1 à 30 pour diminuer le collant, notamment au toucher, d'une composition cosmétique.

50. Utilisation du copolymère selon l'une quelconque des revendications 1 à 30 pour diminuer le collant, notamment au toucher, d'une composition cosmétique dans des conditions d'humidité élevée par exemple de 50 à 100% HR.

51. Utilisation du copolymère selon l'une quelconque des revendications 1 à 30, dans une composition cosmétique telle qu'une composition de soin ou de maquillage pour masquer les rides, qui donne à la peau un aspect lissé, sans tiraillement.

## Claims

1. Block linear ethylene copolymer including:
- at least one block A capable of being prepared from monomers, consisting of a lactam ring-containing ethylene monomer with the following general formula (I): 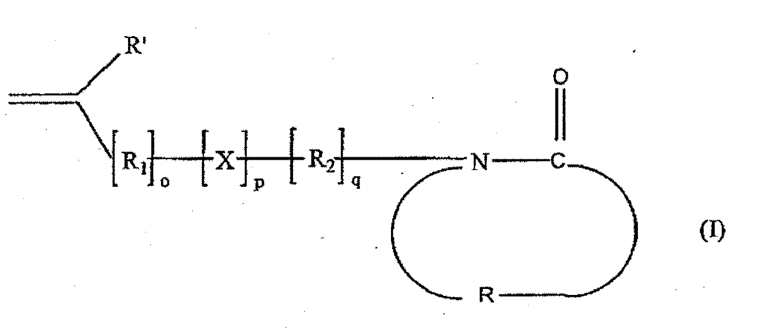 in which:
-- R represents a -(CH₂)ₙ- group in which one or more carbon atoms are optionally replaced by a nitrogen atom or an oxygen atom, where n is an integer from 3 to 12, and where one or more carbon atoms are optionally substituted by one or more C₁ to C₆ alkyl groups;
-- R' represents H or a methyl group;
-- R₁ and R₂, which can be identical or different, represent a linear, branched, or cyclic alkylene or aralkylene group from 1 to 22 C, in which one or more carbon atoms are optionally replaced by an oxygen or nitrogen atom;
-- X is chosen from -OCO-, -NHCO-, -COO-, -0-;
-- o, p and q each represent, independently of one another, 0 or 1;
- and at least one non-hydrophilic monomer, namely a monomer of which the homopolymer prepared from the monomer is not water-soluble or water-dispersible; the percentage of lactam ring-containing ethylene monomer of formula (I) in the block A is 60 to 80% by weight;
- and at least one block B capable of being prepared from monomers not comprising a lactam ring-containing ethylene monomer of formula (I) or comprising a minor proportion thereof.

2. Copolymer according to claim 1, in which, in formula (I), o is 0, p is 1, q is 1, R₂ represents - CH₂CH₂-, X represents COO or CONH, and R is (CH₂)₃ or (CH₂) ₅ or (CH₂CH₂NH) .

3. Copolymer according to claim 1, in which the lactam ring-containing ethylene monomer is a vinyl lactam with the following formula (II): 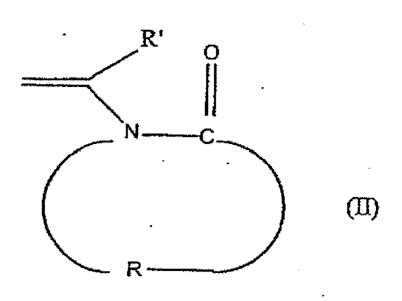 in which R and R' have the meaning already given in claim 1.

4. Copolymer according to any one of claims 1 to 3, in which, in formulas (I) and (II), R represents - (CH₂)ₙ-, where n is an integer of 3 to 5, or R represents -CH₂CH₂-NH-.

5. Copolymer according to any one of the preceding claims, which is a film-forming copolymer.

6. Copolymer according to any one of the preceding claims, which has a number-average molecular weight of 4,000 to 1,000,000, preferably 4,000 to 800,000, and more preferably 4,000 to 500,000.

7. Copolymer according to any one of the preceding claims, in which the block A represents 1 to 99% by weight of the copolymer, preferably 10 to 95%, and more preferably 20 to 90% by weight of the total weight of the copolymer.

8. Copolymer according to any one of the preceding claims, in which the block B represents 1 to 99% by weight of the copolymer, preferably 5 to 90%, and more preferably 10 to 80% by weight of the total weight of the copolymer.

9. Copolymer according to any one of the preceding claims, in which the molecular weight of each block A or B is between 2,000 and 1,000,000, preferably 2,000 and 800,000, and more preferably 2,000 and 500,000.

10. Copolymer according to any one of the preceding claims, in which said monomers from which said block A is prepared include a lactam ring-, for example, vinyl lactam-containing ethylene monomer of formula (I) or (II), in an amount from 65 to 70% by weight, for example 70% by weight.

11. Copolymer according to any one of the preceding claims, in which said block A has a overall glass transition temperature of the block between 0 and 250°C, preferably between 0 and 220°C and more preferably between 5 and 200°C.

12. Copolymer according to claim 1, in which said N-vinyl lactam of formula (I) is pyrrolidinoethyl acrylate or pyrrolidinoethyl methacrylate.

13. Copolymer according to any one of claims 3 to 12, in which said N-vinyl lactam of formula (II) is N-vinylpyrrolidone (n=3), N-vinylpiperidinone (valerolactam) (n=4), N-vinylcaprolactam (n=5), N-vinylimidazolidinone in which R is the -CH₂-CH₂-NH' group, N-vinyl-5-methyl-2-pyrrolidone, N-vinyl-5-ethyl-2-pyrrolidone, N-vinyl-6-methyl-2-piperidone, N-vinyl-6-ethyl-2-piperidone, N-vinyl-7-methyl-2-caprolactam, or N-vinyl-7-ethyl-2-caprolactam.

14. Copolymer according to any one of the preceding claims, in which block A is a random, alternate or gradient copolymer.

15. Copolymer according to any one of the preceding claims, in which said non-hydrophilic monomers of block A are chosen from the following monomers:
- The ethylene hydrocarbons with 2 to 10 C, such as ethylene, isoprene or butadiene;
- The acrylates of formula CH₂=CHCOOR₃;
- The methacrylates of formula: 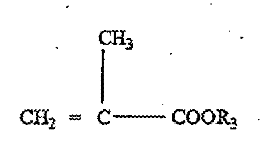 in which R₃ represents:
-- a linear or branched alkyl group with 1 to 18 carbon atoms, in which one or more heteroatoms chosen from 0, N, S and P (is) are optionally inserted,
which alkyl group may also optionally be substituted by one or more substituents chosen from the hydroxyl groups, the halogen atoms (Cl, Br, I and F), and the Si groups (R₄R₅), where R₄ and R₅, identical or different, represent a C₁ to C₆ alkyl group or a phenyl group,
-- a C₃ to C₁₂ cycloalkyl group, such as the isobornyl group,
-- a C₃ to C₂₀ aryl group, such as the phenyl group,
-- a C₄ to C₃₀ aralkyl group (C₁ to C₈ alkyl group) such as 2-phenyl-ethyl or benzyl,
-- a heterocyclic group with 4 to 12 members containing one or more heteroatoms chosen from O, N and S, in which the ring is aromatic or not,
-- a heterocyclylalkyl (alkyl with 1 to 4 C) such as furfurylmethyl or tetrahydrofurfurylmethyl,
in which said cycloalkyl, aryl, aralkyl, heterocyclic or heterocyclylalkyl groups may optionally be substituted by one or more substituents chosen from the hydroxyl groups, the halogen atoms, and the linear or branched alkyl groups with 1 to 4 C in which one or more heteroatoms chosen from 0, N, S and P are optionally inserted, which alkyl groups may also optionally be substituted by one or more substituents chosen from the hydroxyl groups, the halogen atoms (Cl, Br, I and F), and the Si (R₄R₅) groups, where R₄ and R₅, identical or different, represent a C₁ to C₆ alkyl group or a phenyl group,
-- examples of R₃ groups are the methyl, ethyl, propyl, isobutyl, n-butyl, tertiobutyl, hexyl, ethylhexyl, octyl, lauryl, isoctyl, isodecyl, dodecyl, cyclohexyl, t-butylcyclohexyl, t-butylbenzyl, isobornyl, phenyl, furfurylmethyl, tetrahydrofurfurylmethyl and ethyl-2-perfluorohexyl groups,
-- another example of R₃ group for the acrylates are the groups R₃ = - (OC₂H₄) ₘ-OR" , with m=5 to 150 and R"=H or C₆ to C₃₀ alkyl, for example lauryl -POE-;
- The (meth)acrylamides of formula: 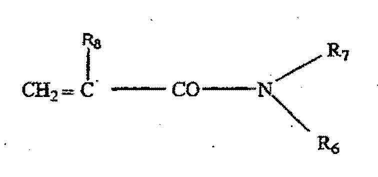 where
R₈ designates H or methyl,
and R₇ and R₆, identical or different, each represent a hydrogen atom or a linear or branched alkyl group with 1 to 18 carbon atoms, in which one or more heteroatoms chosen from 0, N, S and P are optionally inserted, which alkyl groups may also optionally be substituted by one or more substituents chosen from the hydroxyl groups, the halogen atoms (Cl, Br, I and F), and the Si (R₄R₅) groups, where R₄ and R₅, identical or different, represent a C₁ to C₆ alkyl group or a phenyl group,
examples of these groups are the methyl, ethyl, n-butyl, t-butyl, isopropyl, isohexyl, isooctyl and isononyl groups,
-- a C₃ to C₁₂ cycloalkyl group, such as the isobornyl group,
-- a C₃ to C₂₀ aryl group, such as phenyl,
-- a C₄ to C₃₀ aralkyl group (C₁ to C₈ alkyl group) such as 2-phenyl-ethyl or benzyl,
-- a heterocyclic group with 4 to 12 members containing one or more heteroatoms chosen from O, N and S, in which the ring is aromatic or not,
-- a heterocyclylalkyl (alkyl with 1 to 4 C) such as furfurylmethyl or tetrahydrofurfurylmethyl,
in which said cycloalkyl, aryl, aralkyl, heterocyclic or heterocyclylalkyl groups may optionally be substituted by one or more substituents chosen from the hydroxyl groups, the halogen atoms, and the linear or branched alkyl groups with 1 to 4 C in which one or more heteroatoms chosen from O, N, S and P are optionally inserted, which alkyl groups may also optionally be substituted by one or more substituents chosen from the hydroxyl groups, the halogen atoms (Cl, Br, I and F), and the Si (R₄R₅) groups, where R₄ and R₅, identical or different, represent a C₁ to C₆ alkyl group or a phenyl group,
examples of (meth)acrylamide monomers are N-ethyl(meth)acrylamide, N-butylacrylamide, N-t-butylacrylamide, N-isopropylacrylamide, N,N-dimethyl(meth)acrylamide, N-octylacrylamide, N-dodecylacrylamide, and undecylacrylamide;
- The allyl compounds of formula:
CH₂=CH-CH₂-R₉ or CH₂=C(CH₃)-CH₂-R₉;
- The vinyl compounds of formula:
CH₂=CH-R₉,
where R₉ is a group:
-- OR₁₀, where R₁₀ represents a phenyl group or a C₁ to C₁₂ alkyl group (the monomer is a vinyl or allyl ether),
-- OCOR₁₁, where R₁₁ represents:
--- a linear or branched alkyl group with 2 to 12 C (the monomer is a vinyl or allyl ester);
--- a C₃ to C₁₂ cycloalkyl group, such as isobornyl or cyclohexyl,
--- a C₃ to C₂₀ aryl group, such as phenyl,
--- a C₄ to C₃₀ aralkyl group (C₁ to C₈ alkyl group) such as 2-phenylethyl or benzyl,
--- a heterocyclic group with 4 to 12 members containing one or more heteroatoms chosen from 0, N and S, in which the ring is aromatic or not,
--- a heterocyclylalkyl (alkyl with 1 to 4 C) such as furfurylmethyl or tetrahydrofurfurylmethyl,
in which said cycloalkyl, aryl, aralkyl, heterocyclic or heterocyclylalkyl groups may optionally be substituted by one or more substituents chosen from the hydroxyl groups, the halogen atoms, and the linear or branched alkyl groups with 1 to 4 C in which one or more heteroatoms chosen from 0, N, S and P are optionally inserted, which alkyl groups may also optionally be substituted by one or more substituents chosen from the hydroxyl groups, the halogen atoms (Cl, Br, I and F), and the Si (R₄R₅) groups, where R₄ and R₅, identical or different, represent a C₁ to C₆ alkyl group or a phenyl group,
examples of vinyl monomers are vinylcyclohexane and styrene,
examples of vinyl esters are: vinyl acetate, vinyl propionate, vinyl butyrate, vinyl ethylhexanoate, vinyl neononanoate and vinyl neododecanoate, and the vinyl ethers include, for example, vinyl methyl ether, vinyl ethyl ether and vinyl isobutyl ether;
- The (meth)acrylic or (meth)acrylamide or vinyl monomers with a fluorinated or perfluorinated group, such as perfluorooctyl ethyl methacrylate;
- The silicon-containing vinyl or (meth)acrylic monomers, such as methacryloxypropyltris(trimethylsiloxy)silane, acryloxypropylpolydimethylsiloxane, or the silicon-containing (meth)acrylamides.

16. Copolymer according to any one of the preceding claims, in which the monomer(s) other than the lactam-ring-containing monomer from which block A can be prepared is (are) preferably chosen from the monomers of which the glass transition temperature Tg of the corresponding homopolymer is low, preferably less than or equal to 50°C, more preferably less than or equal to 20°C and even more preferably less than or equal to 0°C .

17. Copolymer according to claim 16, in which the monomer(s) other than the lactam ring-containing ethylene monomer further have a Tg of the corresponding homopolymer greater-than or equal to -150°C.

18. Copolymer according to claim 15, in which the monomer(s) other than the lactam ring-containing ethylene monomer from which block A can be prepared is (are) chosen from the following monomers, of which the glass transition temperature Tg of the corresponding homopolymer is less than or equal to 50°C, such as: methyl acrylate (Tg=10°C), ethyl acrylate (Tg=-24°C), n-butyl acrylate (Tg=-54°C), t-butyl acrylate (Tg=43°C), ethylhexyl acrylate (Tg=-50°C), isobutyl acrylate (-24°C), butyl methacrylate (Tg=20°C), n-hexyl methacrylate, POE methacrylate (n=8 to 10) (Tg=-55°C), vinyl acetate (Tg=23°C); and other monomers, such as methyl methacrylate (Tg=105°C), ethyl methacrylate, isobutyl methacrylate, furfuryl acrylate, isobornyl acrylate, tertiobutylcyclohexyl acrylate, styrene and vinylcyclohexane.

19. Copolymer according to any one of the preceding claims, in which the block(s) B other than block A is (are) capable of being prepared from the ethylene monomer(s) chosen from: the allyl monomers, the acrylates, the methacrylates, the acrylamides, the methacrylamides, the vinyl monomers and mixtures thereof, and optionally lactam ring-containing ethylene monomers, for example vinyl lactam monomers, of formula (I) or (II), in which the weight proportion of said monomers (I) or (II) is less than 50% by weight, preferably less than or equal to 45% by weight, more preferably less than or equal to 40% by weight, and even more preferably less than or equal to 30% by weight.

20. Copolymer according to any one of the preceding claims, in which said monomer(s) from which block B can be prepared is (are) chosen from the non-hydrophilic monomers and the hydrophilic monomers.

21. Copolymer according to any one of the preceding claims, in which block B has a glass transition temperature of less than or equal to 50°C, preferably less than or equal to 20°C, and even more preferably less than or equal to 0°C.

22. Copolymer according to any one of the preceding claims, in which the monomer(s) from which the block(s) B can be prepared is (are) chosen from the monomers of which the glass transition temperature Tg of the corresponding homopolymer is low, preferably less than or equal to 50°C, more preferably less than or equal to 20°C, and even more preferably less than or equal to 0°C.

23. Copolymer according to claim 22, in which said monomer(s) further have a Tg of the corresponding homopolymer greater-than -150°C.

24. Copolymer according to claim 20, in which the hydrophilic monomers are chosen from the ionic monomers such as the cationic monomers, the anionic monomers and the betaines; the non-ionic monomers; and the monomers capable of being made hydrophilic by hydrolysis.

25. Copolymer according to claim 20, in which the non-hydrophilic monomers are chosen from the monomers as defined in claims 15 to 18.

26. Copolymer according to claim 24, in which the cationic monomers are chosen from 2-vinylpyridine; 4-vinylpyridine, dimethylaminoethyl (meth)acrylate; diethylaminoethyl (meth)acrylate; dimethylaminopropyl (meth)acrylamide; and the salified or quaternized forms thereof, whether they are mineral acids, such as sulfuric acid or hydrochloric acid, or organic acid salts.

27. Copolymer according to claim 24, in which the anionic monomers are chosen from acrylic acid, methacrylic acid, crotonic acid, maleic anhydride, itaconic acid, fumaric acid, maleic acid; styrenesulfonic acid, 2-acrylamido-2-methylpropanesulfonic acid, vinylsulfonic acid, vinylbenzoic acid, sulfopropyl methacrylate, vinylphosphonic acid and the salts thereof.

28. Copolymer according to claim 24, in which the betaine-type ionic monomers are chosen from:
- ethylene carboxybetaine or sulfobetaines obtained, for example, by quaternization of monomers with ethylene unsaturation comprising an amine function by carboxylic acid salts with mobile halogen, for example, sodium chloroacetate, or by cyclic sulfones, for example propanesulfone.

29. Copolymer according to claim 24, in which the non-ionic monomers are chosen from:
- hydroxyalkyl (meth)acrylates or (metha)crylamides of which the alkyl group has 2 to 4 C atoms, in particular hydroxymethyl or hydroxypropyl (meth)acrylate;
- alkoxyl (C₁₋₄) alkyl (C₁₋₄) (meth)acrylates or (metha)crylamides such as methoxyethyl, ethoxyethyl and methoxypropyl,
- (meth)acrylates or (metha)crylamides with a - (OC₂H₄)ₘ-OR'''-group, with m=5 to 150 and, R'''=H or C₁ to C₄ alkyl, for example methoxy -POE-; -POE-OH;
- vinyl lactams,
- and polysaccharide (meth)acrylates such as saccharose acrylate.

30. Copolymer according to any one of the preceding claims, chosen from:
- the diblock copolymers (AB);
- the triblock copolymers (ABA, BAB, ABC, ACB), with C being different from A or B,
- the multiblock copolymers having more than three blocks: (AB)ₙ, (ABA)ₙ, (BAB)ₙ, (ABC)ₙ, (ACB)ₙ, with C being different from A or B or the multiblock copolymers having more than three different blocks, of the ABCD type.

31. Cosmetic or pharmaceutical composition including the copolymer according to any one of claims 1 to 30.

32. Composition according to claim 31, containing from 0.1 to 60% by weight, preferably 0.5 to 50% by weight, and even more preferably 1 to 40% by weight of the copolymer.

33. Composition according to either one of claims 31 and 32, including, aside from said copolymer, a physiologically acceptable medium, in which the copolymer is in dissolved or dispersed form.

34. Composition according to any one of claims 31 to 33, in which the physiologically acceptable medium includes one or more suitable solvents forming a hydrophilic phase chosen from water and the mixtures of water and hydrophilic organic solvent(s), such as alcohols and in particular lower linear or branched monoalcohols having 2 to 5 carbon atoms such as ethanol, isopropanol and n-propanol, and polyols such as glycerine, diglycerine, propylene glycol, sorbitol, penthylene glycol and the polyethylene glycols.

35. Composition according to claim 34, in which the hydrophilic phase also contains C₂ ethers.

36. Composition according to any one of claims 31 to 35, in which said physiologically acceptable medium also includes a fatty phase, in particular consisting of liquid fats at room temperature, and/or solid fats at room temperature, of animal, plant, mineral or synthetic origin.

37. Composition according to any one of claims 31 to 36, in which the physiologically acceptable medium also includes one or more cosmetically and/or pharmaceutically acceptable organic solvents.

38. Composition according to any one of claims 31 to 37, in which said physiologically acceptable medium also includes one or more auxiliary film-forming agents chosen from the plasticizing agents and the coalescence agents.

39. Composition according to any one of claims 31 to 38, in which the physiologically acceptable medium also includes one or more coloring agents chosen from the water-soluble coloring agents, and the powdered coloring agents, such as pigments, mother-of-pearl and flakes.

40. Composition according to any one of claims 31 to 39, in which the physiologically acceptable medium also includes one or more fillers.

41. Composition according to any one of claims 31 to 40, in which the physiologically acceptable medium also includes one or more ingredient(s) commonly used in cosmetics and/or pharmacy, such as vitamins, thickeners, trace elements, softeners, sequestering agents, fragrances, alkalizing or acidifying agents, preservatives, sunscreens, surfactants, antioxidants, anti-hair loss agents, anti-dandruff agents, propellants, water-soluble or fat-soluble polymers or polymers dispersed in water or in an aqueous phase, fibrogenic or not, and mixtures thereof.

42. Composition according to any one of claims 31 to 41, **characterized in that** it is in the form of a suspension, a dispersion, a solution, a gel, an emulsion, in particular an oil-in-water (O/W) or water-in-oil (W/O) or multiple (W/O/W or polyol/O/W or O/W/O) emulsion, in the form of a cream, a paste, a foam, a dispersion of vesicles, in particular ionic lipids or not, a biphase or multiphase lotion, a spray, a powder, a paste, in particular a flexible paste or an anhydrous paste.

43. Composition according to any one of claims 31 to 42, **characterized in that** it is a hair product, such as a lacquer or a shampoo.

44. Composition according to any one of claims 31 to 42, **characterized in that** it is a make-up composition, such as a nail polish.

45. Keratin material cosmetic make-up or care process including the application of a composition according to one of claims 31 to 44 on the keratin material.

46. Use of the copolymer according to any one of claims 1 to 30 in order to improve hair styling power and/or hold-out, without stickiness, in particular to the touch, of a hair lacquer.

47. Use of the copolymer according to any one of claims 1 to 30, in order to increase the adhesion and wear resistance, without stickiness, in particular to the touch, of a nail polish.

48. Use of the copolymer according to any one of claims 1 to 30, in order to improve the hold-out of a make-up composition.

49. Use of the copolymer according to any one of claims 1 to 30, in order to reduce the stickiness, in particular to the touch, of a cosmetic composition.

50. Use of the copolymer according to any one of claims 1 to 30, in order to reduce the stickiness, in particular to the touch, of a cosmetic composition, under very humid conditions, for example 50 to 100% RH.

51. Use of the copolymer according to any one of claims 1 to 30, in a cosmetic composition such as a care or make-up composition for hiding wrinkles, which gives the skin a smooth appearance without pulling.

## Patentansprüche

1. Lineares ethylenisches Sequenzcopolymer enthaltend:
- mindestens eine Sequenz A, die ausgehend von Monomeren herstellbar ist, die aus einem ethylenischen Monomer mit Lactamring der folgenden Formel (I); 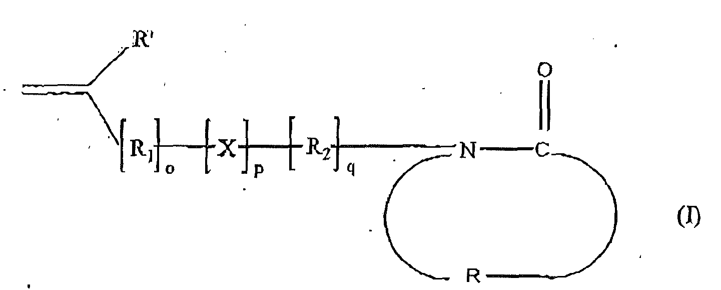 worin:
• R eine Gruppe -(CH₂)ₙ- bedeutet, wobei ein oder mehrere Kohlenstoffatome gegebenenfalls durch ein Stickstoffatom oder ein Sauerstoffatom ersetzt sein können, n eine ganze Zahl von 3 bis 12 bedeutet und ein oder mehrere Kohlenstoffatome gegebenenfalls mit einer oder mehreren C₁₋₆-Alkylgruppen substituiert sind;
• R' H oder die Methylgruppe bedeutet;
• R₁ und R₂, die gleich oder verschieden sein können, eine geradkettige, verzweigte oder cyclische Alkylen- oder Aralkylengruppe mit 1 bis 22 C bedeuten, worin ein oder mehrere Kohlenstoffatome gegebenenfalls durch ein Sauerstoffatom oder Stickstoffatom ersetzt sind;
• X unter -OCO-, -NHCO-, -COO- oder -O- ausgewählt ist;
• o, p und q jeweils unabhängig voneinander 0 oder 1 bedeuten;
und mindestens einem nicht hydrophilen Monomer aufgebaut sind, d.h, einem Monomer, dessen Homopolymer ausgehend von diesem Monomer hergestellt wird, das nicht wasserlöslich und nicht in Wasser dispergierbar ist; wobei der prozentuale Anteil des ethylenischen Monomers mit Lactam-ring der Formel (I) in der Sequenz A 60 bis 80 Gew.-% beträgt;
- und mindestens eine Sequenz B, die aus Monomeren herstellbar ist, die kein ethylenisches Monomer mit Lactamring der Formel (I) oder einen geringeren Anteil dieses Monomers enthalten.

2. Copolymer nach Anspruch 1, wobei in der Formel (I) o 0 bedeutet, p 1 ist, q 1 ist, R₂ -CH₂CH₂- bedeutet; X COO oder CONH bedeutet und R (CH₂)₃ oder (CH₂)₅ oder (CH₂CH₂NH) ist.

3. Copolymer nach Anspruch 1, wobei das ethylenische Monomer mit Lactamring ein Vinyllactam ist, das der folgenden Formel (II) entspricht: 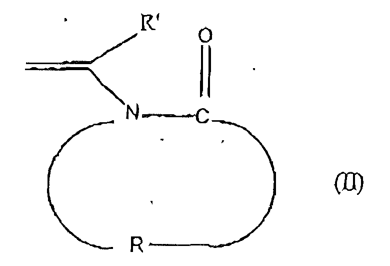 worin R und R' die in Anspruch 1 angegebenen Bedeutungen aufweisen.

4. Copolymer nach einem der Ansprüche 1 bis 3, wobei in den Formeln (I) und (II) die Gruppe R -(CH₂)ₙ-, wobei n eine ganze Zahl von 3 bis 5 ist, oder die Gruppe R -CH₂-CH₂-NH- bedeutet.

5. Copolymer nach einem der vorhergehenden Ansprüche, bei dem es sich um ein filmbildendes Copolymer handelt.

6. Copolymer nach einem der vorhergehenden Ansprüche, das eine zahlenmittlere Molmasse von 4 000 bis 1 000 000, vorzugsweise 4 000 bis 800 000 und noch bevorzugter 4 000 bis 500 000 aufweist.

7. Copolymer nach einem der vorhergehenden Ansprüche, bei dem die Sequenz A 1 bis 99 Gew.-% des Copolymers, vorzugsweise 10 bis 95 Gew.-% und noch bevorzugter 20 bis 90 Gew.-% des Gesamtgewichts des Copolymer ausmacht.

8. Copolymer nach einem der vorhergehenden Ansprüche, bei dem die Sequenz B 1 bis 99 Gew.-% des Copolymers , vorzugsweise 5 bis 90 Gew.-% und noch bevorzugter 10 bis 80 Gew.-% des Gesamtgewichts des Copolymers ausmacht.

9. Copolymer nach einem der vorhergehenden Ansprüche, bei dem die Molmasse jeder Sequenz A oder B im Bereich von 2 000 bis 1 000 000, vorzugsweise 2 000 bis 800 000 und noch bevorzugter 2 000 bis 500 000 liegt.

10. Copolymer nach einem der vorhergehenden Ansprüche, bei dem die Monomere, aus denen die Sequenz A hergestellt wird, einen Anteil des ethylenischen Monomers mit Lactamring, z.B. eines Vinyllactams, der Formel (I) oder (II), im Bereich von 65 bis 75 Gew.-% und beispielsweise 70 Gew.-% umfassen.

11. Copolymer nach einem der vorhergehenden Ansprüche, bei dem die Sequenz A eine Gesamtglasübergangstemperatur der Sequenz im Bereich von 0 bis 250 °C, vorzugsweise 0 bis 220 °C und noch bevorzugter im Bereich von 5 bis 200 °C aufweist.

12. Copolymer nach Anspruch 1, bei dem das N-Vinyllactam der Formel (I) das Pyrrilidinoethylacrylat oder Pyrrilidinoethylmethacrylat ist.

13. Copolymer nach einem der Ansprüche 3 bis 12, bei dem das N-Vinyllactam der Formel (II) das N-Vinylpyrrolidon (n=3), N-Vinylpiperidinon (Valerolactam) (n=4), N-Vinylcaprolactam (n=5), N-Vinylimidazolidinon, bei dem R die Gruppe -CH₂-CH₂-NH- bedeutet, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam oder N-Vinyl-7-ethyl-2-caprolactam ist.

14. Copolymer nach einem der vorhergehenden Ansprüche, wobei die Sequenz A ein statistisches Copolymer, ein alternierendes Copolymer oder ein Gradientencopolymer ist.

15. Copolymer nach einem der vorhergehenden Ansprüche, wobei die nicht hydrophilen Monomere der Sequenz A unter den folgenden Monomeren ausgewählt sind:
- ethylenisch ungesättigten Kohlenwasserstoffen mit 2 bis 10 C, wie Ethylen, Isopren oder Butadien;
- Acrylaten der Formel CH₂=CHCOOR₃;
- Methacrylaten der Formel: 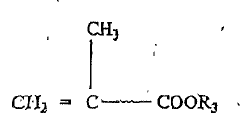 worin R₃ bedeutet:
• eine geradkettige oder verzweigte Alkylgruppe mit 1 bis 18 Kohlenstoffatomen, in die gegebenenfalls ein oder mehrere Heteroatome, die unter O, N, S und P ausgewählt sind, eingebaut sind,
wobei die Alkylgruppe gegebenenfalls ferner mit einem oder mehreren Substituenten substituiert sein kann, die unter Hydroxy, Halogenatomen (C1, Br, 1 und F) und Si(R₄R₅) ausgewählt sind, wobei R₄ und R₅, die gleich oder verschieden sind, eine C₁₋₆-Alkylgruppe oder eine Phenylgruppe bedeuten,
• eine Cycloalkylgruppe mit 3 bis 12 Kohlenstoffatomen, wie Isobornyl,
• eine Arylgruppe mit 3 bis 20 Kohlenstoffatomen, wie Phenyl,
• eine Aralkylgruppe mit 4 bis 30 Kohlenstoffatomen (C₁₋₈-Alkylgruppe), beispielsweise 2-Phenylethyl oder Benzyl,
• eine heterocyclische Gruppe mit 4 bis 12 Bestandteilen, die ein oder mehrere Heteroatome enthält, die unter O, N und S ausgewählt sind, wobei der Ring aromatisch oder nichtaromatisch sein kann,
• eine Heterocyclylalkylgruppe (Alkyl mit 1 bis 4 C), wie Furfurylmethyl oder Tetrahydrofurfurylmethyl,
wobei die Cycloalkylgruppen, Arylgruppen, Aralkylgruppen, heterocyclischen Gruppen oder Heterocyclylalkylgruppen gegebenenfalls mit einem oder mehreren Substituenten substituiert sein können, die unter Hydroxy, Halogenatomen und geradkettigen oder verzweigten C₁₋₄-Alkylgruppen ausgewählt sind, in die gegebenenfalls ein oder mehrere Heteroatome, die unter O, N, S und P ausgewählt sind, eingebaut sein können, wobei die Alkylgruppen ferner gegebenenfalls mit einem oder mehreren Substituenten substituiert sein können, die unter Hydroxy, Halogenatomen (C1, Br, I und F) und Si(R₄R₅) ausgewählt sind, wobei R₄ und R₅, die gleich oder verschieden sind, C₁₋₆-Alkyl oder Phenyl bedeuten,
wobei Beispiele für Gruppen R₃ sind: Methyl, Ethyl, Propyl, Isobutyl, *n*-Butyl, *t*-Butyl, Hexyl, Ethylhexyl, Octyl, Lauryl, Isooctyl, Isodecyl, Dodecyl, Cyclohexyl, *tert*-Butylcyclohexyl, *tert*-Butylbenzyl, Isobornyl, Phenyl, Furfurylmethyl, Tetrahydrofurfurylmethyl, 2-Ethylperfluorhexyl,
• ein weiteres Beispiel für die Gruppe R₃ der Acrylate sind Gruppen R₃ = -(OC₂H₄)ₘ-OR" mit m = 5 bis 150 und R" = H oder C₆₋₃₀-Alkyl, beispielsweise -PEO-Lauryl;
- (Meth)acrylamiden der Formel: 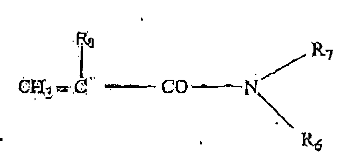 worin
R₈ H oder Methyl bedeutet;
und R₇ und R₆, die gleich oder verschieden sind, jeweils ein Wasserstoffatom oder eine geradkettige oder verzweigte Alkylgruppe mit 1 bis 18 Kohlenstoffatomen bedeuten, in die gegebenenfalls ein oder mehrere Heteroatome, die unter O, N, S und P ausgewählt sind, eingebaut sind, und wobei die Alkylgruppe ferner gegebenenfalls mit einem oder mehreren Substituenten substituiert sein kann, die unter Hydroxygruppen, Halogenatomen (C1, Br, I und F) und Gruppen Si(R₄R₅) ausgewählt sind, wobei R₄ und R₅, die gleich oder verschieden sind, C₁₋₆-Alkyl oder Phenyl bedeuten,
wobei Beispiele für diese Gruppen Methyl, Ethyl, *n*-Butyl, *tert*-Butyl, Isopropyl, Isohexyl, Isooctyl und Isononyl sind,
• Cycloalkyl mit 3 bis 12 Kohlenstoffatomen, wie Isobornyl,
• C₃₋₂₀-Aryl, wie Phenyl,
• Aralkyl mit 4 bis 30 Kohlenstoffatomen (Alkyl mit 1 bis 8 Kohlenstoffatomen), wie 2-Phenylethyl oder Benzyl,
• eine heterocyclische Gruppe mit 4 bis 12 Bestandteilen, die ein oder mehrere Heteroatome enthält, die unter O, N und S ausgewählt sind, wobei der Ring aromatisch oder nichtaromatisch ist,
• eine Heterocyclylalkylgruppe (Alkyl mit 1 bis 4 C), wie beispielsweise Furfurylmethyl oder Tetrahydrofurfurylmethyl,
wobei die Cycloalkylgruppen, Arylgruppen, Aralkylgruppen, heterocyclischen Gruppen oder Heterocyclylalkylgruppen gegebenenfalls mit einem oder mehreren Substituenten substituiert sein können, der (die) unter den Hydroxygruppen, Halogenatomen und geradkettigen oder verzweigten Alkylgruppen mit 1 bis 4 C ausgewählt sind, in die gegebenenfalls ein oder mehrere Heteroatome, die unter O, N, S und P ausgewählt sind, eingebaut sind, wobei die Alkylgruppen ferner gegebenenfalls mit einem oder mehreren Substituenten substituiert sind, die unter den Hydroxygruppen, Halogenatomen (C1, Br, I und F) und den Gruppen Si(R₄R₅) ausgewählt sind, wobei R₄ und R₅, die gleich oder verschieden sind, eine C₁₋₆-Alkylgruppe oder Phenyl bedeuten,
wobei Beispiele für (Meth)acrylamidmonomere N-Ethyl-(meth)acrylamid, N-Butylacrylamid, N-*tert*-Butylacrylamid, N-Isopropylacrylamid, N,N-Dimethyl(meth)acrylamid, N-Octylacrylamid, N-Dodecylacrylamid und Undecylacrylamid sind;
- Allylverbindungen der Formel: CH₂ = CH-CH₂-R₉ oder CH₂ = C(CH₃)-CH₂-R₉;
- Vinylverbindungen der Formel: CH₂ = CH-R₉, worin R₉ bedeutet:
- OR₁₀, wobei R₁₀ eine Phenylgruppe oder eine C₁₋₁₂-Alkylgruppe ist (das Monomer ist ein Vinylether oder Allylether),
- OCOR₁₁, wobei R₁₁ bedeutet:
• eine geradkettige oder verzweigte Alkylgruppe mit 2 bis 12 C (das Monomer ist ein Vinylester oder Allylester);
• eine Cycloalkylgruppe mit 3 bis 12 Kohlenstoffatomen, wie Isobornyl, Cyclohexyl,
• eine Arylgruppe mit 3 bis 20 Kohlenstoffatomen, wie Phenyl,
• eine Aralkylgruppe mit 4 bis 30 Kohlenstoffatomen (Alkylgruppe mit 1 bis 8 C), wie 2-Phenylethyl, Benzyl,
• eine heterocyclische Gruppe mit 4 bis 12 Bestandteilen, die ein oder mehrere Heteroatome enthält, die unter O, N und S ausgewählt sind, wobei der Ring aromatisch oder nichtaromatisch sein kann,
• eine Heterocyclylalkylgruppe (Alkyl mit 1 bis 4 C), wie beispielsweise Furfurylmethyl oder Tetrahydrofurfurylmethyl,
wobei die Cycloalkylgruppen, Arylgruppen, Aralkylgruppen, heterocyclischen Gruppen oder Heterocyclylalkylgruppen gegebenenfalls mit einem oder mehreren Substituenten substituiert sein können, der unter den Hydroxygruppen, Halogenatomen und geradkettigen oder verzweigten Alkylgruppen mit 1 bis 4 C ausgewählt sind, in die gegebenenfalls ein oder mehrere Heteroatome eingebaut sind, die unter O, N, S und P ausgewählt sind, wobei die Alkylgruppen ferner gegebenenfalls mit einem oder mehreren Substituenten substituiert sein können, die unter Hydroxygruppen, Halogenatomen (C1, Br, I und F) und Gruppen Si(R₄R₅) ausgewählt sind, wobei R₄ und R₅, die gleich oder verschieden sind, C₁₋₆-Alkyl oder Phenyl bedeuten,
wobei Beispiele für Vinylmonomere Vinylcyclohexan und Styrol sind,
Beispiele für Vinylester sind; Vinylacetat, Vinylpropionat, Vinylbutyrat, Vinylethylhexanoat, Vinylneononanoat und Vinylneododecanoat,
von den Vinylestern sind beispielsweise Vinylmethylether, Vinylethylether und Vinylisobutylether zu nennen;
- (Meth)acrylmonomeren oder (Meth)acrylamiden oder Vinylmonomeren mit fluorierter oder perfluorierter Gruppe, wie Ethylperfluoroctylmethacrylat;
- (Meth)acrylamonomeren oder Vinylmonomeren, die siliconiert sind, wie Methacryloxypropyltris(trimethylsiloxy)silan, Acryloxypropylpolydimethylsiloxan oder (Meth)acrylamiden, die siliconiert sind.

16. Copolymer nach einem der vorhergehenden Ansprüche, wobei das oder die Monomer(e), die von dem ethylenischen Monomer mit Lactamring verschieden sind und ausgehend von dem oder denen die Sequenz A herstellbar ist, vorzugsweise unter den Monomeren ausgewählt sind, deren Glasübergangstemperatur Tg des entsprechenden Homopolymers niedrig ist, vorzugsweise kleiner oder gleich 50 °C ist, besser kleiner oder gleich 20 °C ist und noch besser kleiner oder gleich 10 °C beträgt.

17. Copolymer nach Anspruch 16, bei dem das oder die Monomer(e), die von dem ethylenischen Monomer mit Lactamring verschieden sind, ferner eine Tg des entsprechenden Homopolymers aufweisen, die größer oder gleich -150 °C ist.

18. Copolymer nach Anspruch 15, bei dem das oder die Monomer(e), die von dem ethylenischen Monomer mit Lactamring verschieden sind und ausgehend von dem oder denen die Sequenz A herstellbar ist, unter den folgenden Monomeren ausgewählt ist (sind), bei denen die Glasübergangstemperatur Tg des entsprechenden Homopolymers höchstens 50 °C beträgt, wie Methylacrylat (Tg = 10 °C), Ethylacrylat (Tg = -24 °C), n-Butylacrylat (Tg = -54 °C), *tert*-Butylacrylat (Tg = 43 °C), Ethylhexylacrylat (Tg = -50 °C), Isobutylacrylat (-24 °C), Butylmethacrylat (Tg = 20 °C), n-Hexylmethacrylat, PEO-Methacrylat (n = 8 bis 10) (Tg = -55 °C) Vinylacetat (Tg = 23 °C); und weiteren Monomeren, wie Methylmethacrylat (Tg = 105 °C), Ethylmethacrylat, Isobutylmethacrylat, Furfurylacrylat, Isobornylacrylat, tert-Butylcyclohexylacrylat, Styrol und Vinylcyclohexan,

19. Copolymer nach einem der vorhergehenden Ansprüche, wobei die von der Sequenz A verschiedene(n) Sequenz(en) B aus einem oder mehreren ethylenisch ungesättigten Monomeren herstellbar ist (sind), die ausgewählt sind unter: Allylmonomeren, Acrylaten, Methacrylaten, Acrylamiden, Methacrylamiden, Vinylmonomeren und deren Gemischen und gegebenenfalls ethylenisch ungesättigten Monomeren mit Lactamring, beispielsweise Vinyllactammonomeren, der Formel (I) oder (II), wobei der Gewichtsanteil der Monomere (I) oder (II) unter 50 Gew.-% liegt und bevorzugt kleiner oder gleich 45 Gew.-% und vorzugsweise kleiner oder gleich 40 Gew.-% und noch bevorzugter kleiner oder gleich 30 Gew.-% ist.

20. Copolymer nach einem der vorhergehenden Ansprüche, bei dem das oder die Monomer(e), ausgehend von dem oder denen die Sequenz B herstellbar ist, unter den nicht hydrophilen Monomeren und unter den hydrophilen Monomeren ausgewählt ist (sind).

21. Copolymer nach einem der vorhergehenden Ansprüche, wobei die Sequenz B eine Glasübergangstemperatur Tg aufweist, die kleiner oder gleich 50 °C ist, vorzugsweise kleiner oder gleich 20 °C und noch besser kleiner oder gleich 0 °C ist.

22. Copolymer nach einem der vorhergehenden Ansprüche, wobei das oder die Monomer(e), ausgehend von dem oder denen die Sequenz(en) B herstellbar ist (sind), unter den Monomeren ausgewählt sind, deren Glasübergangstemperatur Tg des entsprechenden Homopolymers niedrig und vorzugsweise kleiner oder gleich 50 °C ist, besser kleiner oder gleich 20 °C ist und noch besser kleiner oder gleich 0 °C beträgt.

23. Copolymer nach Anspruch 22, bei dem das oder die Monomer(e) ferner eine Tg des entsprechenden Homopolymers aufweisen, die größer oder gleich -150 °C ist.

24. Copolymer nach Anspruch 20, bei dem die hydrophilen Monomere unter den ionischen Monomeren, wie kationischen Monomeren, anionischen Monomeren und Betainen; nichtionischen Monomeren; und den Monomeren ausgewählt sind, die infolge einer Hydrolyse hydrophil gemacht werden können.

25. Copolymer nach Anspruch 20, bei dem die nicht hydrophilen Monomere unter den Monomeren ausgewählt sind, wie sie in den Ansprüchen 15 bis 18 definiert sind.

26. Copolymer nach Anspruch 24, wobei die kationischen Monomere unter 2-Vinylpyridin; 4-Vinylpyridin, Dimethylaminoethyl(meth)-acrylat; Diethylaminoethyl(meth)acrylat; Dimethylaminopropyl(meth)acrylamid und den in Form der Salze vorliegenden Formen oder quaternisierten Formen dieser Monomere ausgewählt sind, wobei es sich um Salze anorganischer Säuren, wie Schwefelsäure oder Salzsäure, oder Salze organischer Säuren handelt.

27. Copolymer nach Anspruch 24, wobei die anionischen Monomere unter Acrylsäure, Methacrylsäure, Crotonsäure, Maleinsäureanhydrid, Itaconsäure, Fumarsäure, Maleinsäure, Styrolsulfonsäure, 2-Acrylamido-2-methylpropansulfonsäure, Vinylsulfonsäure, Vinylbenzoesäure, Sulfopropylmethacrylat, Vinylphosphonsäure, und den Salzen dieser Monomere ausgewählt sind.

28. Copolymer nach Anspruch 24, wobei die ionischen Monomere vom Betaintyp ausgewählt sind unter:
- ethylenisch ungesättigten Carboxybetainen oder Sulfobetainen, die beispielsweise durch Quaternisierung von Monomeren mit ethylenisch ungesättigter Bindung, die eine Aminfunktion enthalten, mit Carbonsäuresalzen mit mobilem Halogen, beispielsweise Natriumchloracetat, oder mit cyclischen Sulfonen, beispielsweise Propansulfon, erhalten werden.

29. Copolymer nach Anspruch 24, wobei die nichtionischen Monomere ausgewählt sind unter:
- Hydroxyalkyl(meth)acrylaten oder Hydroxyalkyl(meth)acrylamiden, bei denen die Alkylgruppe 2 bis 4 C-Atome aufweist, insbesondere Hydroxyethyl(meth)acrylat oder Hydroxypropyl(meth)acrylat,
- Alkoxy(C₁₋₄)alkyl(C₁₋₄)(meth)acrylaten oder -(meth)acrylamiden, beispielsweise Methoxyethyl(meth)acrylat oder
- (meth)acrylamid, Ethoxyethyl(meth)acrylat oder -(meth)-acrylamid oder Methoxypropyl(meth)acrylat oder -(meth)-acrylamid,
- (Meth)acrylaten oder (Meth)acrylamiden mit einer Gruppe -(OC₂H₄)ₘ-OR'" mit m = 5 bis 150 und R'" = H oder C₁₋₄-Alkyl, beispielsweise -PEO-Methoxy, -PEO-OH;
- Vinyllactamen; und
- (Meth)acrylaten von Polysacchariden, wie Saccharoseacrylat.

30. Copolymer nach einem der vorhergehenden Ansprüche, das ausgewählt ist unter:
- den bisequentiellen Copolymeren (AB);
- trisequentiellen Copolymeren (ABA, BAB, ABC, ACB), wobei die Sequenz C von den Sequenzen A und B verschieden ist;
- multisequentiellen Copolymeren mit mehr als 3 Sequenzen; (AB)n, (ABA)n, (BAB)n, (ABC)n, wobei C von A oder B verschieden ist, oder multisequentiellen Copolymeren vom Typ ABCD, die mehr als drei unterschiedliche Sequenzen aufweisen.

31. Kosmetische oder pharmazeutische Zusammensetzung, die das Copolymer nach einem der Ansprüche 1 bis 30 enthält.

32. Zusammensetzung nach Anspruch 31, die 0,1 bis 60 Gew.-%, vorzugsweise 0,5 bis 50 Gew.-% und noch bevorzugter 1 bis 40 Gew.-% des Copolymers enthält.

33. Zusammensetzung nach einem der Ansprüche 31 und 32, die neben dem Copolymer ein physiologisch akzeptables Medium aufweist, in dem das Copolymer in gelöster oder dispergierter Form vorliegt.

34. Zusammensetzung nach einem der Ansprüche 31 bis 33, wobei das physiologisch akzeptable Medium ein oder mehrere geeignete Lösungsmittel enthält, die eine hydrophile Phase bilden und die unter Wasser und Gemischen von Wasser und einem oder mehreren hydrophilen organischen Lösungsmitteln ausgewählt sind, wie Alkoholen und insbesondere geradkettigen oder verzweigten niederen Monoalkoholen mit 2 bis 5 Kohlenstoffatomen, beispielsweise Ethanol, Isopropanol oder *n*-Propanol, und Polyolen, wie Glycerin, Diglycerin, Propylenglycol, Sorbit, Pentylenglycol und Polyethylenglycolen.

35. Zusammensetzung nach Anspruch 34, wobei die hydrophile Phase ferner C₂-Ether enthält.

36. Zusammensetzung nach einem der Ansprüche 31 bis 35, wobei das physiologisch akzeptable Medium ferner eine Fettphase umfasst, die aus bei Umgebungstemperatur flüssigen Fettsubstanzen und/oder aus bei Umgebungstemperatur festen Fettsubstanzen tierischer, pflanzlicher, mineralischer oder synthetischer Herkunft zusammengesetzt ist.

37. Zusammensetzung nach einem der Ansprüche 31 bis 36, bei der das physiologisch akzeptable Medium ferner ein oder mehrere kosmetisch und/oder pharmazeutisch akzeptable organische Lösungsmittel enthält.

38. Zusammensetzung nach einem der Ansprüche 31 bis 37, wobei das physiologisch akzeptable Medium ferner ein oder mehrere Hilfsstoffe für die Filmbildung aufweist, die unter den Weichmachern und Koaleszenzmitteln ausgewählt sind.

39. Zusammensetzung nach einem der Ansprüche 31 bis 38, wobei das physiologisch akzeptable Medium ferner ein oder mehrere Farbmittel aufweist, die unter den wasserlöslichen Farbstoffen und pulverförmigen Farbstoffen, wie Pigmenten, Perlglanzstoffen und Pailletten ausgewählt sind.

40. Zusammensetzung nach einem der Ansprüche 31 bis 39, wobei das physiologisch akzeptable Medium ferner Füllstoffe enthält.

41. Zusammensetzung nach einem der Ansprüche 31 bis 40, wobei das physiologisch akzeptable Medium ferner einen oder mehrere Bestandteile enthält, die häufig in der Kosmetik und/oder Pharmazie verwendet werden, wie Vitamine, Verdickungsmittel, Spurenelemente, Weichmacher, Maskierungsmittel, Parfums, Alkalisierungsmittel, Ansäuerungsmittel, Konservierungsmittel, Sonnenschutzfilter, grenzflächenaktive Stoffe, Antioxidantien, Wirkstoffe gegen Haarausfall, Antischuppenmittel, Treibmittel, wasserlösliche Polymere, fettlösliche Polymere oder Polymere in Dispersion in Wasser oder in einer wässrigen Phase, die filmbildend oder nicht filmbildend sind, oder deren Gemische.

42. Zusammensetzung nach einem der Ansprüche 31 bis 41, **dadurch gekennzeichnet, dass** sie als Suspension, Dispersion, Lösung, Gel, Emulsion, insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Spray, Pulver, Paste und insbesondere weiche Paste oder wasserfreie Paste vorliegt.

43. Zusammensetzung nach einem der Ansprüche 31 bis 42, **dadurch gekennzeichnet, dass** es sich um ein Produkt für die Haarbehandlung, beispielsweise einen Haarlack oder ein Haarwaschmittel handelt.

44. Zusammensetzung nach einem der Ansprüche 31 bis 42, **dadurch gekennzeichnet, dass** es sich um eine Zusammensetzung zum Schminken handelt, beispielsweise einen Nagellack.

45. Kosmetisches Verfahren zum Schminken oder für die Pflege von Keratinsubstanzen, das das Aufbringen einer Zusammensetzung nach einem der Ansprüche 31 bis 44 auf die Keratinsubstanzen umfasst.

46. Verwendung des Copolymers nach einem der Ansprüche 1 bis 30, um, ohne zu kleben, die Festigungswirkung und/oder Haftung eines Haarlacks zu verbessern.

47. Verwendung des Copolymers nach einem der Ansprüche 1 bis 30, um die Haftung und Abriebfestigkeit eines Nagellacks zu verbessern, ohne dass dieser insbesondere bei Berührung klebt.

48. Verwendung des Copolymers nach einem der Ansprüche 1 bis 30, um die Haftung einer Zusammensetzung zum Schminken zu verbessern.

49. Verwendung des Copolymers nach einem der Ansprüche 1 bis 30, um die Klebrigkeit einer kosmetischen Zusammensetzung insbesondere bei Berührung zu vermindern.

50. Verwendung des Copolymers nach einem der Ansprüche 1 bis 30, um die Klebrigkeit einer kosmetischen Zusammensetzung insbesondere bei Berührung unter der Bedingung hoher Feuchtigkeit, beispielsweise 50 bis 100 % relativer Feuchte, zu vermindern.

51. Verwendung des Copolymers nach einem der Ansprüche 1 bis 30 in einer kosmetischen Zusammensetzung, beispielsweise einer Zusammensetzung für die Pflege oder zum Schminken, um Falten zu kaschieren, die der Haut, ohne zu spannen, ein glattes Aussehen gibt.
